# EUROPÄISCHE PATENTSCHRIFT

(11) **EP 3 658 112 B1**
(45) Veröffentlichungstag und Bekanntmachung des Hinweises auf die Patenterteilung: **14.07.2021**
(21) Anmeldenummer: 18729362.6
(22) Anmeldetag: 25.05.2018
(51) Int. Cl.: A61K 8/49, A61Q 17/04, A61Q 19/00, A61Q 19/08

(54) **WIRKSTOFFKOMBINATIONEN AUS N-(4-AMINO-2-METHYLCHINOLIN-6-YL)-2-((4-ETHYLPHENOXY)METHYL)BENZAMID UND EINEM ODER MEHREREN KOSMETISCH ODER DERMATOLOGISCH UNBEDENKLICHEN KONSERVIERUNGSMITTELN UND/ODER KONSERVIERUNGSHELFERN**
ACTIVE SUBSTANCE COMBINATIONS OF N-(4-AMINO-2-METHYLQUINOLINE-6-YL)-2-((4-ETHYLPHENOXY)METHYL)BENZAMIDE AND ONE OR MORE COSMETICALLY OR DERMATOLOGICALLY ACCEPTABLE PRESERVATIVES AND/OR PRESERVATIVE AUXILIARIES
ASSOCIATIONS DE PRINCIPES ACTIFS COMPRENANT DU N-(4-AMINO-2-MÉTHYLCHINOLIN-6-YL)-2-((4-ÉTHYLPHÉNOXY)MÉTHYLE)BENZAMIDE ET UN OU PLUSIEURS AGENTS ET/OU ADJUVANTS DE CONSERVATION COSMÉTIQUEMENT OU DERMATOLOGIQUEMENT ACCEPTABLES

(30) Priorität: 24.07.2017 DE 102017212647
(43) Veröffentlichungstag der Anmeldung: 03.06.2020
(73) Patentinhaber: Beiersdorf AG, 20253 Hamburg (DE)
(72) Erfinder: SIEFKEN, Wilfried, 22457 Hamburg (DE); REUTER, Jörn Hendrik, 24558 Henstedt Ulzburg (DE); SKUBSCH, Kerstin, 25497 Prisdorf (DE)
(86) Internationale Anmeldenummer: PCT/EP2018/063797
(87) Internationale Veröffentlichungsnummer: WO 2019/020246

(56) Entgegenhaltungen:
- EP-A1- 2 878 309

## Beschreibung

Die vorliegende Erfindung betrifft kosmetische bzw. dermatologische Zubereitungen, enthaltend N-(4-amino-2-methylchinolin-6-yl)-2-((4-ethylphenoxy)methyl)benzamid zur Pflege und zum Schutze der Haut, insbesondere der empfindlichen Haut wie auch ganz besonders im Vordergrunde stehend der durch intrinsische und/oder extrinsische Faktoren gealterten oder alternden Haut sowie die Verwendung solcher Wirkstoffe und Kombinationen solcher Wirkstoffe auf dem Gebiete der kosmetischen und dermatologischen Hautpflege.

Unter kosmetischer Hautpflege ist in erster Linie zu verstehen, dass die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Bei alter Haut beispielsweise erfolgt die regenerative Erneuerung verlangsamt, wobei insbesondere das Wasserbindungsvermögen der Hornschicht nachläßt. Sie wird deshalb inflexibel, trocken und rissig ("physiologisch" trockene Haut). Ein Barriereschaden ist die Folge. Die Haut wird anfällig für negative Umwelteinflüsse wie die Invasion von Mikroorganismen, Toxinen und Allergenen. Als Folge kann es sogar zu toxischen oder allergischen Hautreaktionen kommen.

Bei pathologisch trockener und empfindlicher Haut liegt ein Barriereschaden a priori vor. Epidermale Interzellularlipide werden fehlerhaft oder in ungenügender Menge bzw. Zusammensetzung gebildet. Die Konsequenz ist eine erhöhte Durchlässigkeit der Hornschicht und ein unzureichender Schutz der Haut vor Verlust an hygroskopischen Substanzen und Wasser.

Die Barrierewirkung der Haut kann über die Bestimmung des transepidermalen Wasserverlustes (TEWL - transepidermal water loss) quantifiziert werden. Dabei handelt es sich um die Abdunstung von Wasser aus dem Körperinneren ohne Einbeziehung des Wasserverlustes beim Schwitzen. Die Bestimmung des TEWL-Wertes hat sich als außerordentlich informativ erwiesen und kann zur Diagnose rissiger oder schrundiger Haut, zur Bestimmung der Verträglichkeit chemisch verschiedenartig aufgebauter Tenside und dergleichen mehr herangezogen werden.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).

Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie zur Behandlung der Folgeschäden der Lichtalterung, insbesondere der unter a) bis g) aufgeführten Phänomene.

Produkte zur Pflege gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Daüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Insbesondere betrifft die vorliegende Erfindung kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

Die vorliegende Erfindung betrifft ferner Antioxidantien, bevorzugt solche, welche in hautpflegenden kosmetischen oder dermatologischen Zubereitungen eingesetzt werden. Insbesondere betrifft die Erfindung auch kosmetische und dermatologische Zubereitungen, solche Antioxidantien enthaltend. In einer bevorzugten Ausführungsform betrifft die vorliegende Erfindung kosmetische und dermatologische Zubereitungen zur Prophylaxe und Behandlung kosmetischer oder dermatologischer Hautveränderungen wie z.B. der Hautalterung, insbesondere der durch oxidative Prozesse hervorgerufenen Hautalterung.

Weiterhin betrifft die vorliegende Erfindung Wirkstoffe und Zubereitungen, solche Wirkstoffe enthaltend, zur kosmetischen und dermatologischen Behandlung oder Prophylaxe erythematöser, entzündlicher, allergischer oder autoimmunreaktiver Erscheinungen, insbesondere Dermatosen.

Die vorliegende Erfindung betrifft in einer weiteren vorteilhaften Ausführungsform Wirkstoffkombinationen und Zubereitungen, die zur Prophylaxe und Behandlung der lichtempfindlichen Haut, insbesondere von Photodermatosen, dienen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, dass UVA-Strahlung zu einer Schädigung der elastischen und kollagenen Fasern des Bindegewebes führt, was die Haut vorzeitig altern läßt, und dass sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)), nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxylradikale, Singulettsauerstoff. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, dass auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzliche Antioxidantien und/oder Radikalfänger einverleibt werden.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der Erfindung war es daher auch, kosmetische, dermatologische und pharmazeutische Wirkstoffe und Zubereitungen sowie Lichtschutzformulierungen zu schaffen, die zur Prophylaxe und Behandlung lichtempfindlicher Haut, insbesondere Photodermatosen, bevorzugt PLD dienen.

Weitere Bezeichnungen für die polymorphe Lichtdermatose sind PLD, PLE, Mallorca-Akne und eine Vielzahl von weiteren Bezeichnungen, wie sie in der Literatur (z.B. A. Voelckel et al, Zentralblatt Haut- und Geschlechtskrankheiten (1989), 156, S.2), angegeben sind.

Hauptsächlich werden Antioxidantien als Schutzsubstanzen gegen den Verderb der sie enthaltenden Zubereitungen verwendet. Dennoch ist bekannt, dass auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs, Frankfurt, und Lester Packer, Berkeley/Californien), werden oxidative Schäden der Haut und ihre näheren Ursachen aufgeführt.

Auch aus dem Grunde, solchen Reaktionen vorzubeugen, können kosmetischen oder dermatologischen Formulierungen zusätzlich Antioxidantien und/oder Radikalfänger einverleibt werden.

Zwar sind einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der vorliegenden Erfindung war es somit, Wege zu finden, die die Nachteile des Standes der Technik vermeiden. Insbesondere soll die Wirkung der Behebung der mit der endogenen, chronologischen und exogenen Hautalterung verbundenen Schäden und die Prophylaxe dauerhaft, nachhaltig und ohne das Risiko von Nebenwirkungen sein. Erfindungsgemäß werden die Übelstände des Standes der Technik beseitigt durch Wirkstoffkombinationen aus N-(4-amino-2-methylchinolin-6-yl)-2-((4-ethylphenoxy)methyl) und einem oder mehreren kosmetisch oder dermatologisch unbedenklichen Konservierungsmitteln oder Konservierungshelfern. Siehe in diesem Zusammenhang insbesondere EP 2 878 309 A1.

Kosmetische oder dermatologische Zubereitungen gemäß der Erfindung, Wirkstoffkombinationen aus N-(4-amino-2-methylchinolin-6-yl)-2-((4-ethylphenoxy)methyl) und einem oder mehreren kosmetisch oder dermatologisch unbedenklichen Konservierungsmitteln oder Konservierungshelfern, sind in jeglicher Hinsicht überaus befriedigende Präparate. Es war für den Fachmann nicht vorauszusehen, dass die Zubereitungen gemäß der Erfindung
- besser die Barriereeigenschaften der Haut erhalten oder wiederherstellen,
- besser der Hautaustrocknung entgegenwirken,
- besser gegen Pigmentstörungen wirken,
- besser gegen die Hautalterung wirken und
- die Haut besser vor Umwelteinflüssen schützen
   als die Zubereitungen des Standes der Technik.

Bei Anwendung der erfindungsgemäß verwendeten Wirkstoffkombinationen bzw. kosmetischer oder topischer dermatologischer Zubereitungen mit einem wirksamen Gehalt an erfindungsgemäß verwendeten Wirkstoffkombinationen ist in überraschender Weise eine wirksame Behandlung, aber auch eine Prophylaxe
- von defizitären, sensitiven oder hypoaktiven Hautzuständen oder defizitären, sensitiven oder hypoaktiven Zustände von Hautanhangsgebilden
- von Erscheinungen vorzeitiger Alterung der Haut (z.B. Falten, Altersflecken, Teleangiektasien) und/oder der Hautanhangsgebilde,
- von umweltbedingten (Rauchen, Smog, reaktive Sauerstoffspecies, freie Radikale) und insbesondere lichtbedingten negativen Veränderungen der Haut und der Hautanhangsgebilde.
- von lichtbedingten Hautschäden
- von Pigmentierungsstörungen,
- von Juckreiz,
- von trockenen Hautzuständen und Hornschichtbarrierestörungen,
- von Haarausfall und für verbessertes Haarwachstum
- von entzündlichen Hautzuständen sowie atopischem Ekzem, seborrhoischem Ekzem, polymorpher Lichtdermatose, Psoriasis, Vitiligo
   möglich. Der erfindungsgemäße Wirkstoffes bzw. kosmetischer oder topischer dermatologischer Zubereitungen mit einem wirksamen Gehalt an erfindungsgemäßem Wirkstoff dient aber auch in überraschender Weise
- zur Beruhigung von empfindlicher oder gereizter Haut
- zur Stimulation der Kollagen-, Hyaluronsäure-, Elastinsynthese
- zur Stimulation der Ceramidsynthese der Haut
- zur Stimulation der intrazellulären DNA-Synthese, insbesondere bei defizitären oder hypoaktiven Hautzuständen.
- zur Steigerung der Zellerneuerung und Regeneration der Haut
- zur Steigerung der hauteigenen Schutz- und Reparaturmechanismen (beispielsweise für dysfunktionelle Enzyme, DNA, Lipide, Proteine)
- zur Vor- und Nachbehandlung bei topischer Anwendung von Laser- und Abschleifbehandlungen, die z. B. der Reduzierung von Hautfalten und Narben dienen, um den resultierenden Hautreizungen entgegenzuwirken und die Regenerationsprozesse in der verletzten Haut zu fördern.

N-(4-amino-2-methylchinolin-6-yl)-2-((4-ethylphenoxy)methyl)benzamid ist gekennzeichnet durch die Strukturformel

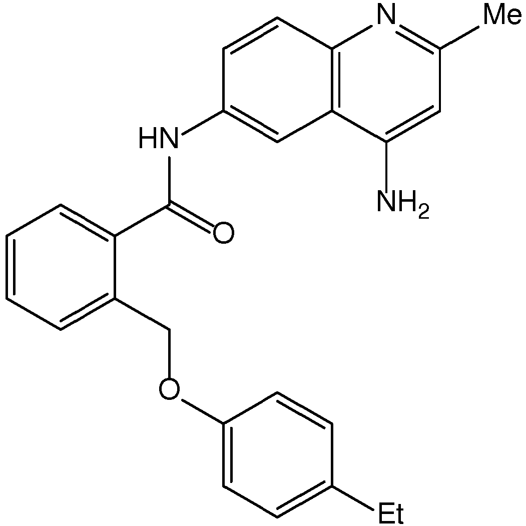

Bevorzugt enthalten kosmetische oder dermatologische Zubereitungen gemäß der Erfindung 0,001 - 10 Gew.-%, besonders bevorzugt 0,01 - 1 Gew.-%, an N-(4-amino-2-methylchinolin-6-yl)-2-((4-ethylphenoxy)methyl)benzamid , bezogen auf die Gesamtzusammensetzung der Zubereitungen.

Vorteilhaft enthalten kosmetische oder dermatologische Zubereitungen gemäß der erfindungsgemäßen Verwendung einen oder mehrere Konservierungsmittel oder Konservierungshelfer, wobei die Gesamtmenge der Konservierungsmittel oder Konservierungshelfer z. B. 0,01 Gew.-% bis 20 Gew.-%, vorzugsweise 0,1 bis 20 Gew.-%, insbesondere 0,5 bis 5,0 Gew.-% beträgt, bezogen auf das Gesamtgewicht der Zubereitungen, um kosmetische Zubereitungen zur Verfügung zu stellen.

Vorteilhaft enthalten die erfindungsgemäßen Zubereitungen 0,01 - 20 Gew-% an einem oder mehreren Konservierungsmitteln oder Konservierungshelfern, vorzugsweise 0,1 -5 Gew-% einem oder mehreren Konservierungsmitteln oder Konservierungshelfern ,insbesondere 4-Hydroxybenzoesäuremethylester, Etylparaben , Phenoxyethanol ,Pirocton-Olamin, Methylisothiazolinon ,Methylpropanediol,Butylene Glycol, Caprylyl Glycol ,Octan-1,2-diol, Ethylhexylglycerin, Glyceryl Caprylate , Propyleneglycol , Natrium Salicylat, Natrium Benzoat,Pentylene Glycol ,1,2-Hexanediol, DMDM Hydantoin, Benzylalkohol, Butylene Glycol, lodopropynyl Butylcarbamate

Ferner ist es erfindungsgemäß vorteilhaft, wenn die Zubereitung Propylenglycol, Butylenglycol, 2-Methylpropan-1,3-diol, Ethylhexylglycerin, 1,2-Pentandiol, 1,2-Hexandiol, 1,2-Octandiol und/oder 1,2-Decandiol enthält.

Dabei ist der Einsatz von Ethylhexylglycerin erfindungsgemäß bevorzugt.

Ethylhexylglycerin wird erfindungsgemäß vorteilhaft in einer Konzentration von 0,1 bis 0,6 Gewichts-%, bezogen auf das Gesamtgewicht der Zubereitung eingesetzt.

Erfindungsgemäß von Vorteil ist auch der Gehalt an Phenoxyethanol in der Zubereitung.

Phenoxyethanol wird erfindungsgemäß vorteilhaft in einer Konzentration von 0,1 bis 1 Gewichts-%, bezogen auf das Gesamtgewicht der Zubereitung eingesetzt.

Erfindungsgemäß bevorzugt ist insbesondere der Einsatz einer Kombination aus Phenoxyethanol und Ethylhexylglycerin.

Die Zubereitungen gemäß der Erfindung enthalten vorteilhaft Substanzen, die UV-Strahlung im UV-A- und/oder UV-B-Bereich absorbieren, wobei die Gesamtmenge der Filtersubstanzen z.B. 0,1 Gew.-% bis 40 Gew.-%, vorzugsweise 1 bis 30 Gew.-%, insbesondere 3 bis 20 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die UV-B-Filter können öllöslich oder wasserlöslich sein. Vorteilhafte öllösliche UV-B-Filtersubstanzen sind z. B.:
▪ 3-Benzylidencampher-Derivate, vorzugsweise 3-(4-Methylbenzyliden)campher, 3-Benzylidencampher;
▪ 4-Aminobenzoesäure-Derivate, vorzugsweise 4-(Dimethylamino)-benzoesäure(2-ethylhexyl)ester, 4-(Dimethylamino)benzoesäureamylester;
▪ 2,4,6-Trianilino-(p-carbo-2'-ethyl-1'-hexyloxy)-1,3,5-triazin;
▪ Ester der Benzalmalonsäure, vorzugsweise 4-Methoxybenzalmalonsäuredi(2-ethylhexyl)ester;
▪ Ester der Zimtsäure, vorzugsweise 4-Methoxyzimtsäure(2-ethylhexyl)ester, 4-Methoxyzimtsäureisopentylester;
▪ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
▪ sowie an Polymere gebundene UV-Filter.

Vorteilhafte wasserlösliche UV-B-Filtersubstanzen sind z. B.:
▪ Salze der 2-Phenylbenzimidazol-5-sulfonsäure, wie ihr Natrium-, Kalium- oder ihr Triethanolammonium-Salz, sowie die Sulfonsäure selbst;
▪ Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Die Liste der genannten UV-Filter, die im Sinne der vorliegenden Erfindung eingesetzt werden können, soll selbstverständlich nicht limitierend sein.

Erfindungsgemäß vorteilhaft können Zubereitungen, welche die erfindungsgemäßen Wirkstoffkombinationen enthalten, übliche Antioxidantien eingesetzt werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Prophylaxe bzw. die kosmetische oder dermatologische Behandlung mit den kosmetischen oder topischen dermatologischen Zubereitungen mit einem wirksamen Gehalt an N-(4-amino-2-methylchinolin-6-yl)-2-((4-ethylphenoxy)methyl)benzamid erfolgt in der üblichen Weise, und zwar dergestalt, dass die kosmetischen oder topischen dermatologischen Zubereitungen mit einem wirksamen Gehalt an N-(4-amino-2-methylchinolin-6-yl)-2-((4-ethylphenoxy)methyl)benzamid auf die betroffenen Hautstellen aufgetragen wird.

Vorteilhaft kann N-(4-amino-2-methylchinolin-6-yl)-2-((4-ethylphenoxy)methyl)benzamid eingearbeitet werden in übliche kosmetische und dermatologische Zubereitungen, welche in verschiedenen Formen vorliegen können. So können sie z.B. eine Lösung, eine Emulsion vom Typ Wasser-in-ÖI (W/O) oder vom Typ Öl-in-Wasser (O/W), oder eine multiple Emulsionen, beispielsweise vom Typ Wasser-in-ÖI-in-Wasser (W/O/W) oder Öl-in-Wasser-in-Öl (O/W/O), eine Hydrodispersion oder Lipodispersion, ein Gel, einen festen Stift oder auch ein Aerosol darstellen.

Erfindungsgemäße Emulsionen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol.

Es ist auch möglich und vorteilhaft im Sinne der vorliegenden Erfindung, den erfindungsgemäß verwendeten Wirkstoff in wäßrige Systeme bzw. Tensidzubereitungen zur Reinigung der Haut und der Haare einzufügen.

Es ist dem Fachmanne natürlich bekannt, dass anspruchsvolle kosmetische Zusammensetzungen zumeist nicht ohne die üblichen Hilfs- und Zusatzstoffe denkbar sind. Die erfindungsgemäßen kosmetischen Zubereitungen können daher kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Verdickungsmittel, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Die Ölphase der Emulsionen der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl, Arganöl, Macadamiaöl, Olus Oil, Shea Butter, Kakaobutter und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Erfindungsgemäße als Emulsionen vorliegenden Zubereitungen enthalten insbesondere vorteilhaft ein oder mehrere Hydrocolloide. Diese Hydrocolloide können vorteilhaft gewählt werden aus der Gruppe der Gummen, Polysaccharide, Cellulosederivate, Schichtsilikate, Polyacrylate, Carbomere, Acrylates/C10-30 Alkyl Acrylate Crosspolymer,und/oder anderen Polymeren.

Erfindungsgemäße als Hydrogele vorliegende Zubereitungen enthalten ein oder mehrere Hydrocolloide. Diese Hydrocolloide können vorteilhaft aus der vorgenannten Gruppe gewählt werden.

### Wirksamkeitstests:

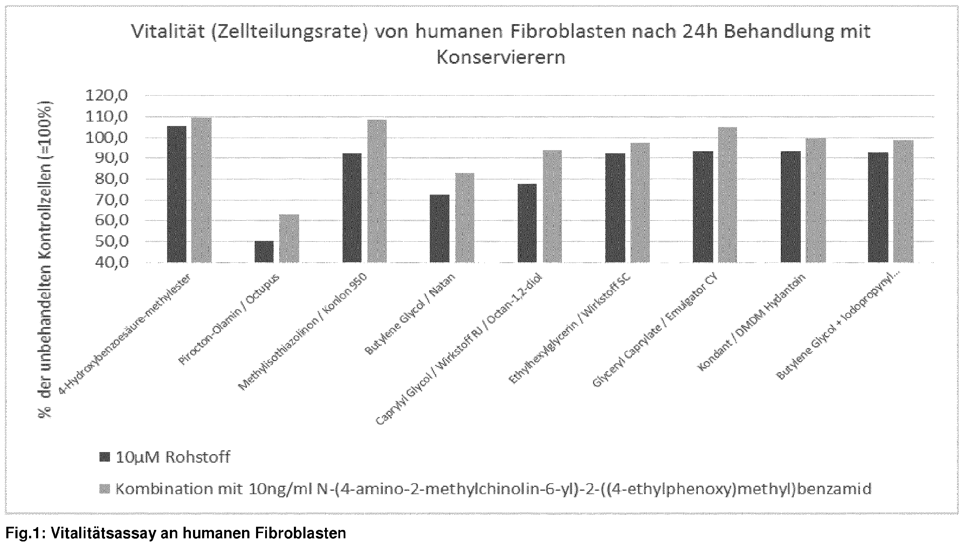

Für den Vitalitätsassay wurden dermale Fibroblasten für 24h mit Wirkstoffen/Wirkstoffkombinationen im Zellkulturmedium behandelt und währenddessen mit BrdU (5-Bromo Deoxy-Uridin) versehen. Die Zellen nehmen diese nichtnatürliche und per Fluoreszenz-Detektion nachweisbare Nucleinsäure auf und bauen sie bei der Zellteilung ins neusynthetisierte Erbgut ein. Somit ist die nachfolgend vermessene Fluoreszenzintensität des Zellrasens nach 24h letztlich ein Maß für die Zellteilungsrate während der Wirkstoffbehandlung. Somit kann auf den Einfluss der jeweiligen Wirkstoffe auf die Zellvitalität zurückgeschlossen werden. Ein positiver Einfluss von N-(4-amino-2-methylchinolin-6-yl)-2-((4-ethylphenoxy)methyl)-benzamid in Kombination mit einem Konservierer zeigt sich dahingehend, dass die Einbaurate von BrdU erhöht gegenüber der alleinigen Behandlung mit dem entsprechenden Konservierer ist, siehe Fig. 1.

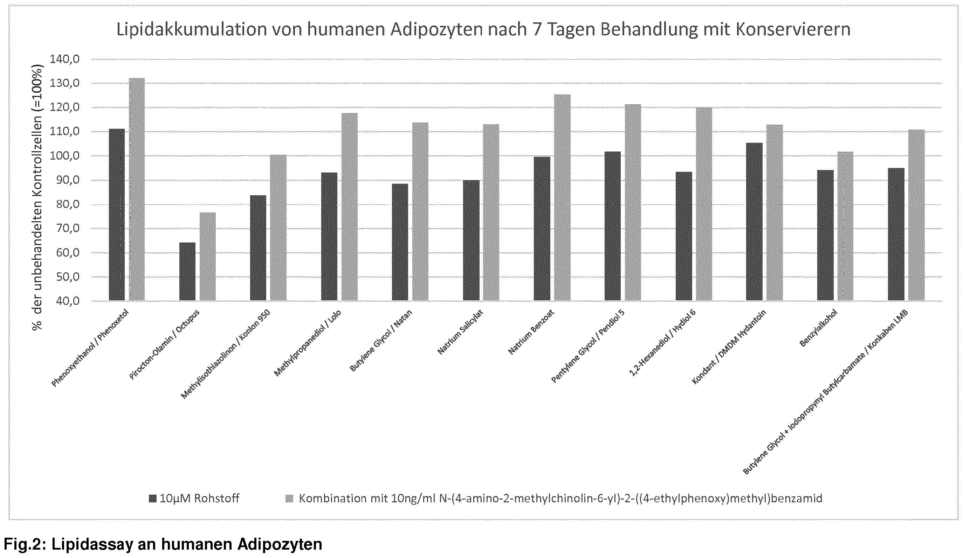

Der Lipidassay wird an subcutanan Adipozyten durchgeführt. Präadipozyten werden in Zellkulturschalen ausgesät und zu Adipozyten differenziert. Hieran schließt sich eine einwöchige Wirkstoffbehandlung an, während der die Zellen Lipide synthetisieren und vesikulär einlagern. Die Gesamtmenge an Lipid in Abhängigkeit von Wirkstoffeffekten wird nach Ablauf der Kulturdauer ermittelt. Hierfür wird der Zellrasen mit dem lipophilen Fluoreszenzfarbstoff AdipoRed angefärbt und anschließend floureszenzspektroskopisch quantifiziert. Ein positiver Einfluss von N-(4-amino-2-methylchinolin-6-yl)-2-((4-ethylphenoxy)methyl)benzamid in Kombination mit einem Konservierer zeigt sich dahingehend, dass die Akkumulation von AdipoRed und mithin die Differenzierungsleistung der Adipozyten erhöht gegenüber der alleinigen Behandlung mit dem entsprechenden Konservierer ist, siehe Fig. 2.

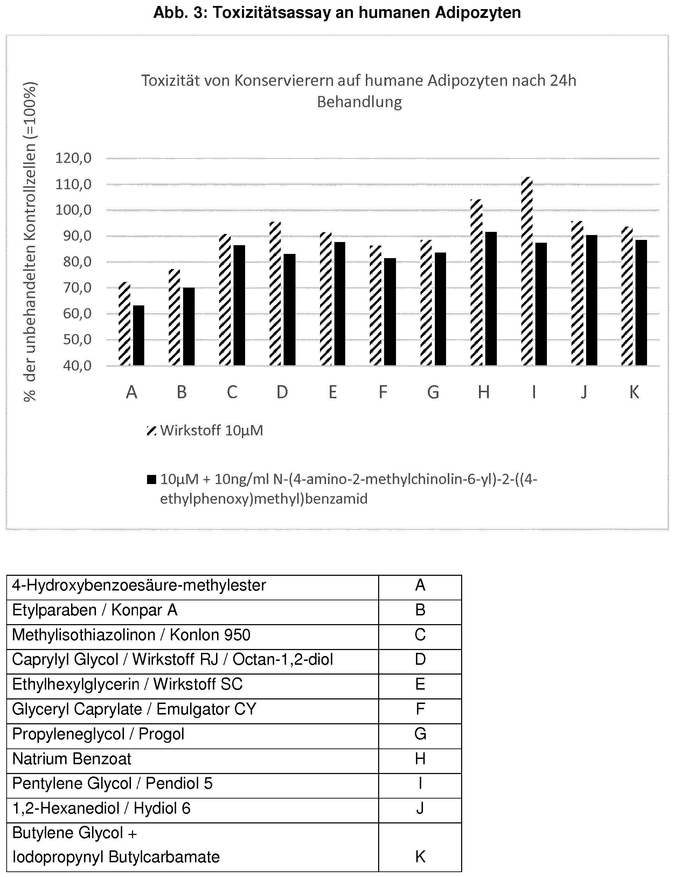

Der dritte durchgeführte Assay ist der LDH (Lactat-Dehydrogenase) Zytotoxizitätsassay. Bei diesem Assay wird aus dem Medienüberstand von Zellrasen das Stoffwechselenzym LDH nachgewiesen, indem der Zellkulturüberstand mit einem Substrat für das Enzym versetzt wird. Nach dessen Umsetzung tritt eine Farbveränderung auf und diese wird photometrisch erfasst. Das Maß der Farbänderung korreliert dabei mit der Menge an LDH im Zellkulturüberstand. LDH ist aber ein intrazelluläres Enzym,und kann nur bei Zellschädigung, z.B. durch toxische Substanzen und gleichzeitiger Schädigung der Zellmembran in den Medienüberstand von Zellkulturen gelangen. Ein positiver Einfluss von N-(4-amino-2-methylchinolin-6-yl)-2-((4-ethylphenoxy)methyl)benzamid in Kombination mit einem Konservierer zeigt sich dahingehend, dass die Austrittsrate von LDH verringert gegenüber der alleinigen Behandlung mit dem entsprechenden Konservierer ist, entsprechend einer verringerten Toxizität der Wirkstoffkambination. Siehe hierzu Fig. 3 für humane Adipozyten:
Für alle Assays lagen die Wirkstoffe als 1000x Stock gelöst in DMSO zu 10mM vor und wurden für den Einsatz entsprechend 1:1000 im jeweiligen Zellkulturmedium verdünnt.

### Tabellarische Zusammenfassung der Ergebnisse:

Für folgende erfindungsgemäße Wirkstoffe wurde für eine getestete Kombination zusammen mit 10ng/ml N-(4-amino-2-methylchinolin-6-yl)-2-((4-ethylphenoxy)methyl)benzamid eine bessere Wirksamkeit in einem oder mehreren Zellkulturassays als für den entsprechenden Wirkstoff alleine beobachtet:

| Wikstoff | | | Assay |
|---|---|---|---|
| 4-Hydroxybenzoesäuremethylester / Konpar M | | | LDH |
| Etylparaben / Konpar A | | | BrdU+LDH |
| Phenoxyethanol / Phenoxetol | | | Lipid |
| Pirocton-Olamin / Octupus | | | Lipid+BrdU |
| Methylisothiazolinon / Konlon 950 | | | Lipid+BrdU |
| Methylpropanediol / Lolo | | | Lipid |
| Butylene Glycol / Natan | | | Lipid+BrdU |
| Caprylyl Glycol / Wirkstoff RJ / Octan-1,2-diol | | | BrdU+LDH |
| Ethylhexylglycerin / Wirkstoff SC | | | BrdU+LDH |
| Glyceryl Caprylate / Emulgator CY | | | BrdU+LDH |
| Propyleneglycol / Progol | | | LDH |
| Natrium Salicylat | | | Lipid |
| Natrium Benzoat | | | Lipid+LDH |
| Pentylene Glycol / Pendiol 5 | | | Lipid+LDH |
| 1,2-Hexanediol / Hydiol 6 | | | Lipid+LDH |
| Kondant / DMDM Hydantoin | | | Lipid+BrdU |
| Benzylalkohol | | | Lipid |
| Butylene Glycol + lodopropynyl Butylcarbamate / Konkaben LMB | | | Lipid+BrdU+LDH |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | **A** | **B** | **C** | **D** |
|---|---|---|---|---|
| **Zusammensetzung in %** | **m [%]** | **m [%]** | **m [%]** | **m [%]** |
| N-(4-amino-2-methylchinolin-6-yl)-2-((4-ethylphenoxy)methyl) | 0,2 | 0,3 | 0,5 | 0,1 |
| Phenoxyethanol | 0,7 | 0,4 | 0,5 | 0,4 |
| Ethylhexylglycerin | | 0,5 | 0,25 | |
| Carnitine | | | 1 | |
| Creatine | | | 2 | |
| Creatinine | | | 0,1 | |
| Coenzym Q10 | | | 0,5 | |
| Arctium Lappa Fruit Extract | | | | 0,2 |
| Tocopherol | | | | 0,1 |
| Dicaprylyl Ether | 3,5 | | | |
| Dimethicone | 0,5 | | | |
| Isopropyl Palmitate | 2 | 9 | | |
| Cera Microcristallina | | 3 | | |
| Paraffinum Liquidum | | 3 | | |
| Isohexadecane | | 2 | | |
| Almond Oil | | 5 | | |
| Shea Butter | | | 3 | |
| C12-15 Alkyl Benzoate | | | 1 | |
| Caprylic/Capric Triglyceride | | | | 2 |
| Hydrogenated Vegetable Oil | | | | 3 |
| Octyldodecanol | | | | 2 |
| Argan Oil | | | | 2 |
| Glyceryl Stearate SE | 2 | | | |
| Sodium Cetearyl Sulfate | 0,3 | | | |
| Stearinsäure | | 2,5 | | |
| Natriumstearoylglutamat | | | 0,5 | |
| Glyceryl Stearate | | | 2 | 1 |
| Glyceryl Stearate Citrate | | | | 1,5 |
| Tapioca Starch | | | 1 | |
| Glycerin | 5 | 20 | 10 | 15 |
| Acrylates/C1 0-30 Alkyl Acrylate Crosspolymer | | 0,3 | | 0,2 |
| Aloe Vera | | 4 | | |
| Panthenol | | | | 1 |
| Urea | | | | 5 |
| Cetearyl Alcohol | 1 | | | |
| Carbomer | 0,2 | | 0,5 | 0,1 |
| Xanthan Gum | | 0,1 | | 0,2 |
| Alcohol Denat | 2 | | | |
| Calcium Chloride | | | 0,1 | |
| Natronlauge q.s. auf pH Wert | pH 7,0 | pH 6,5 | pH 6,5 | pH 5,5 |
| Wasser | ad 100 | ad 100 | ad 100 | ad 100 |

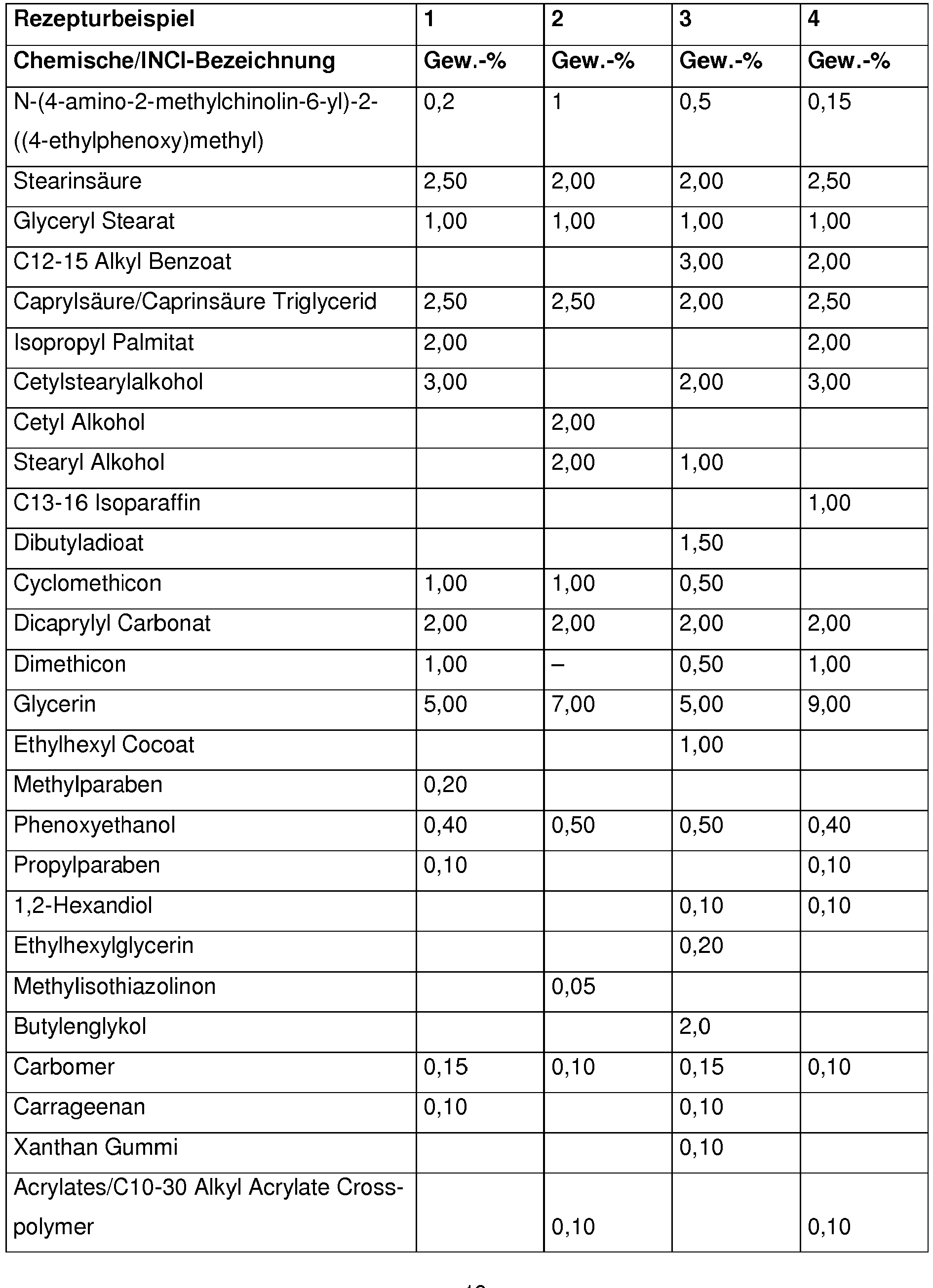

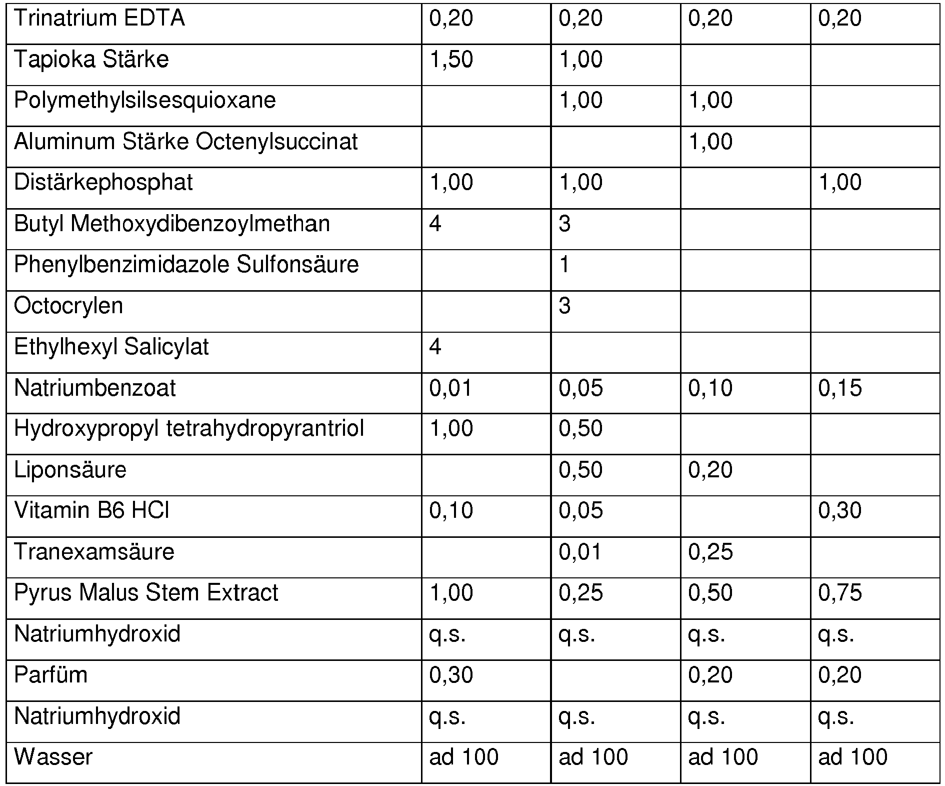

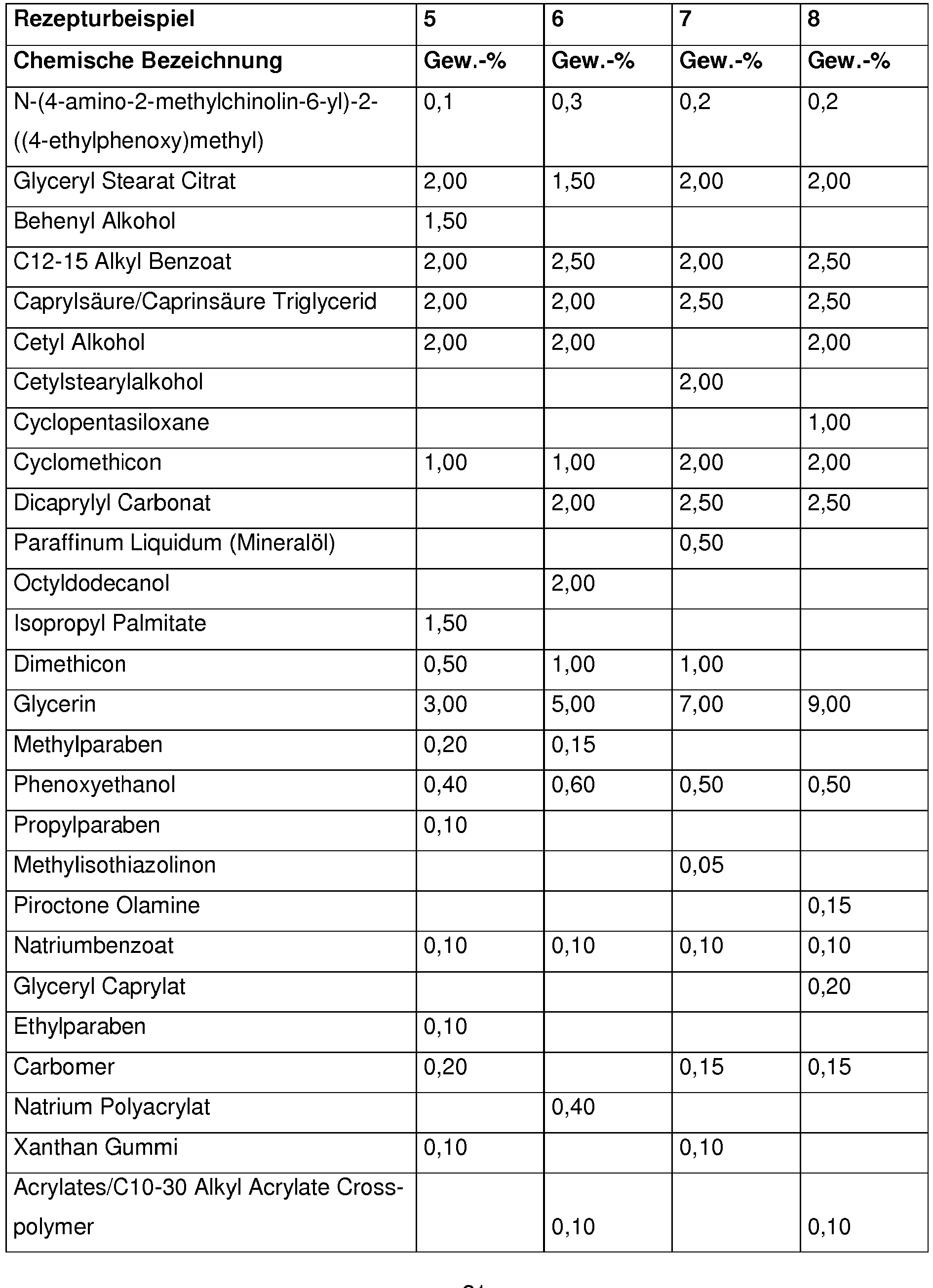

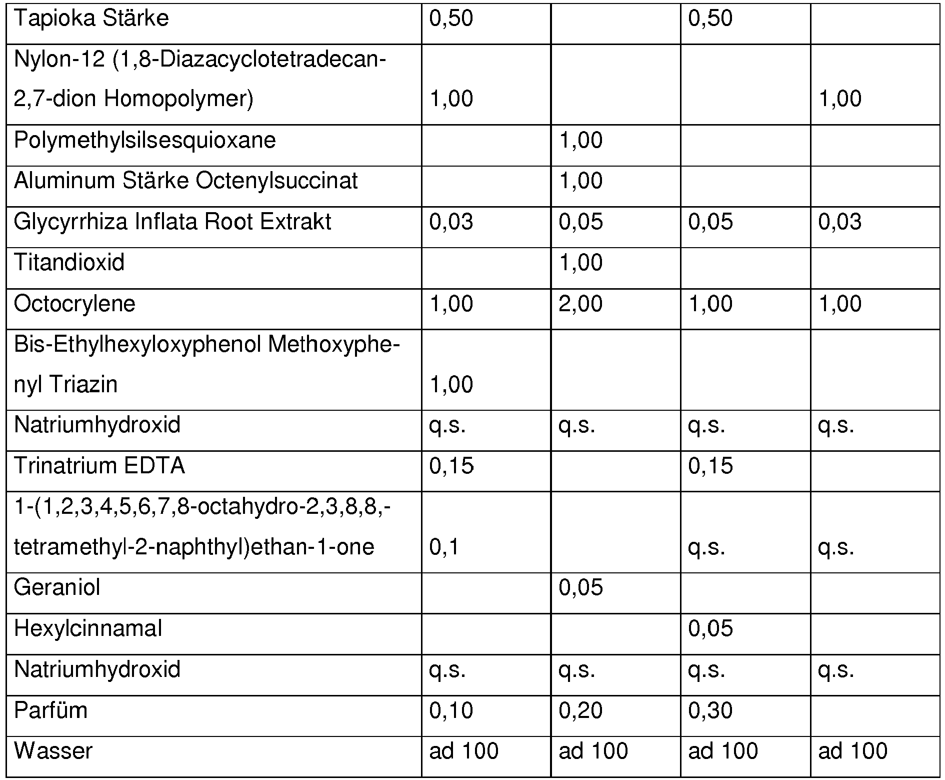

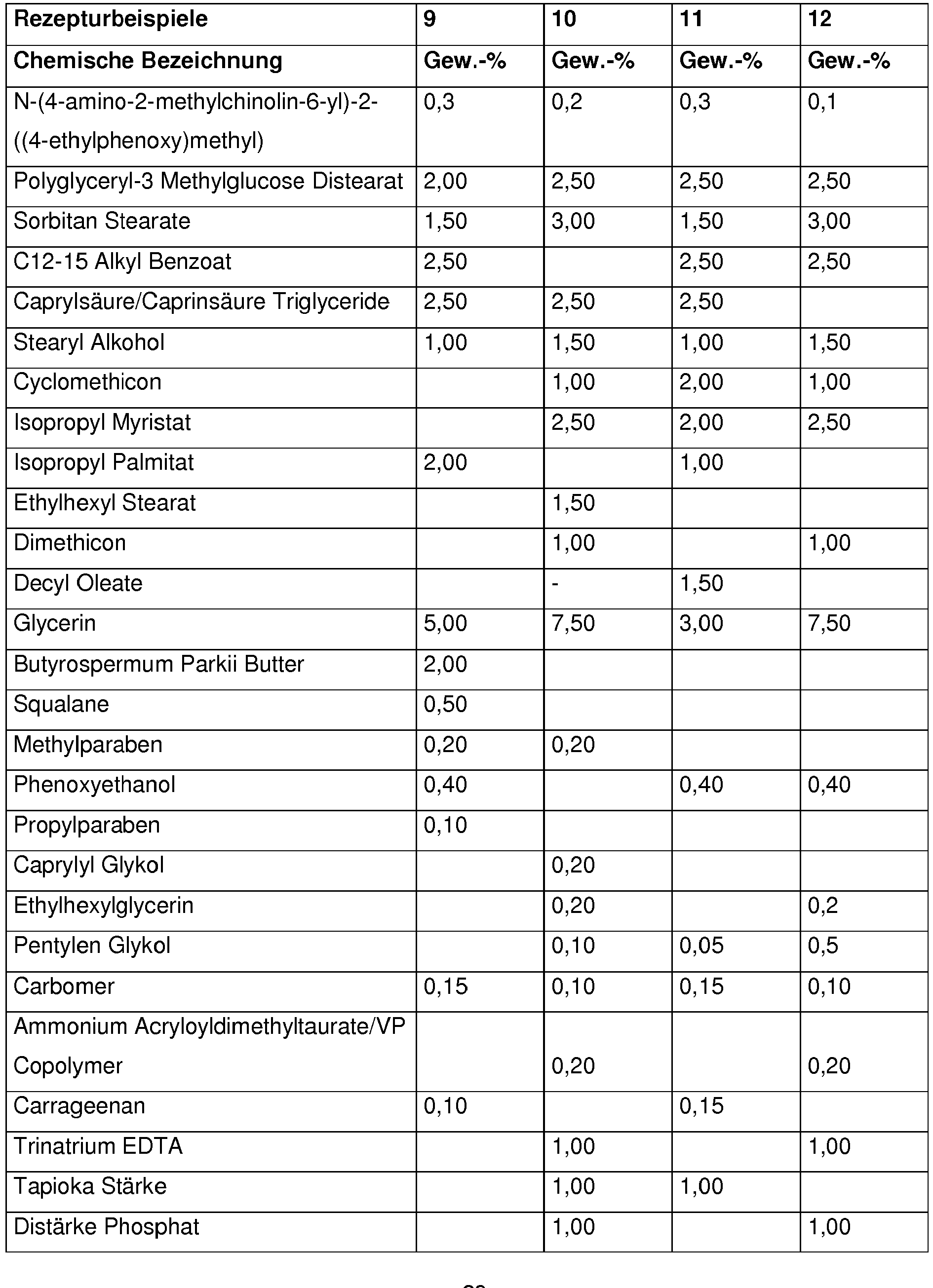

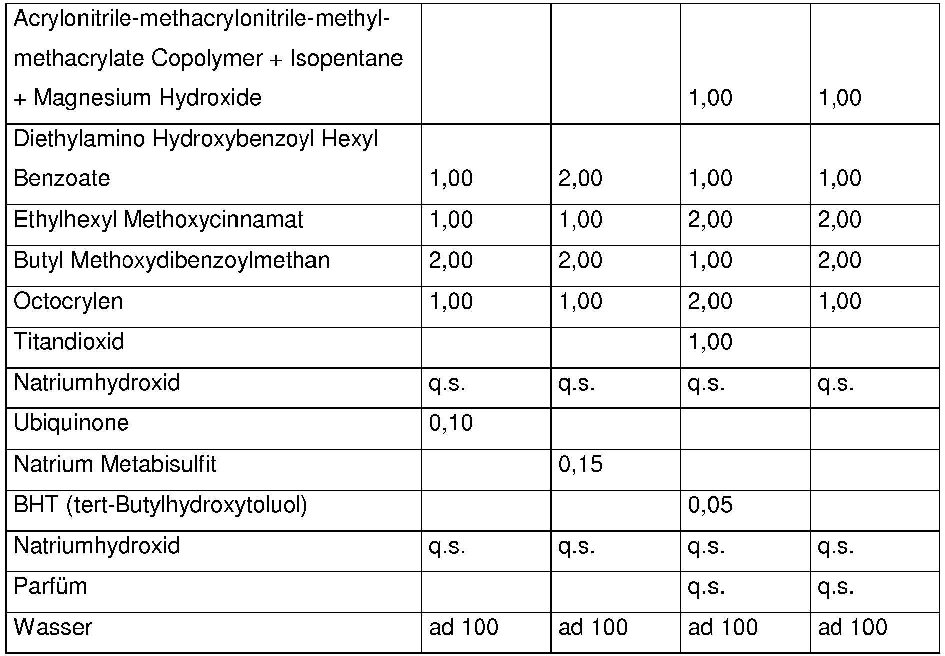

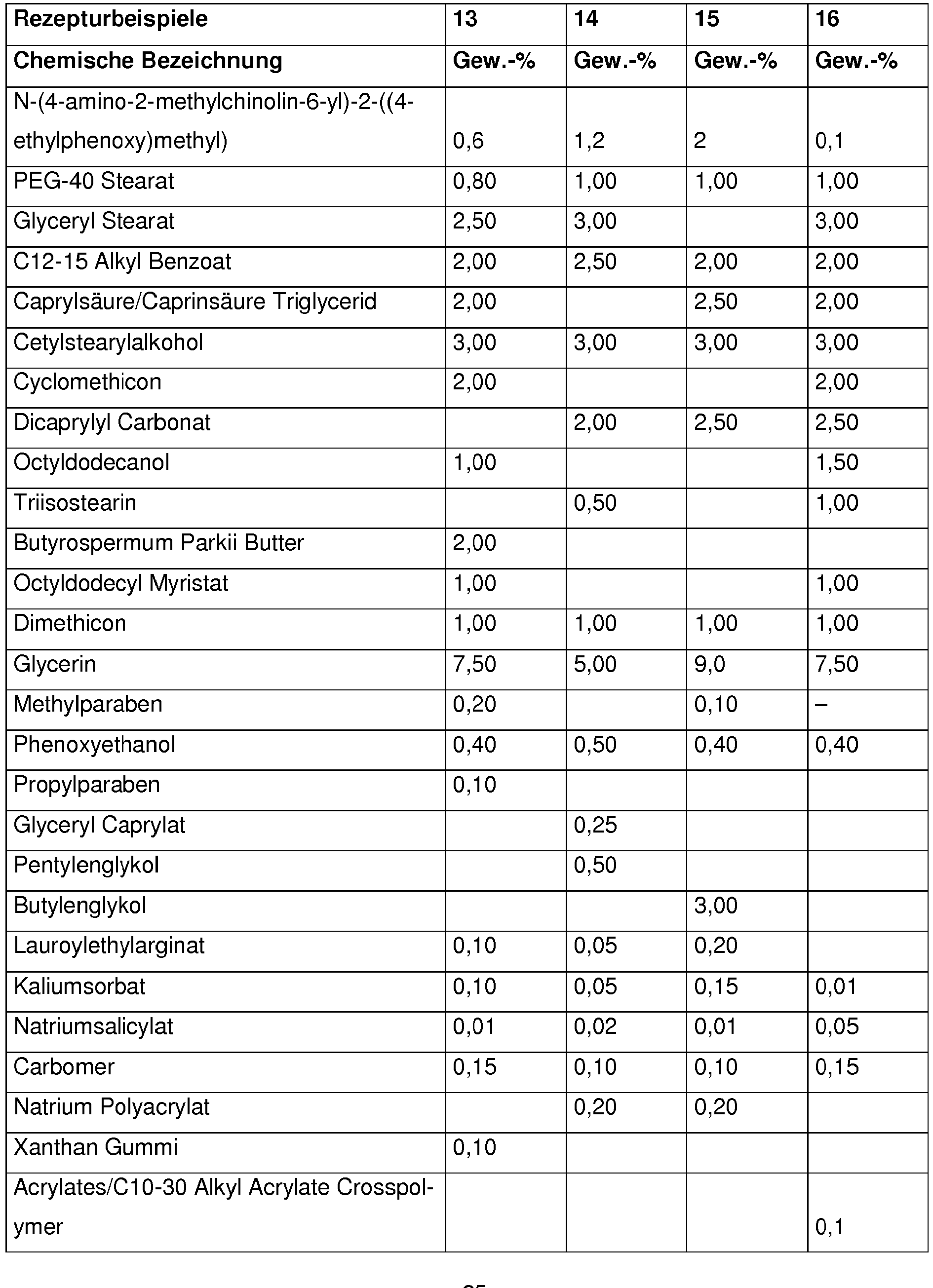

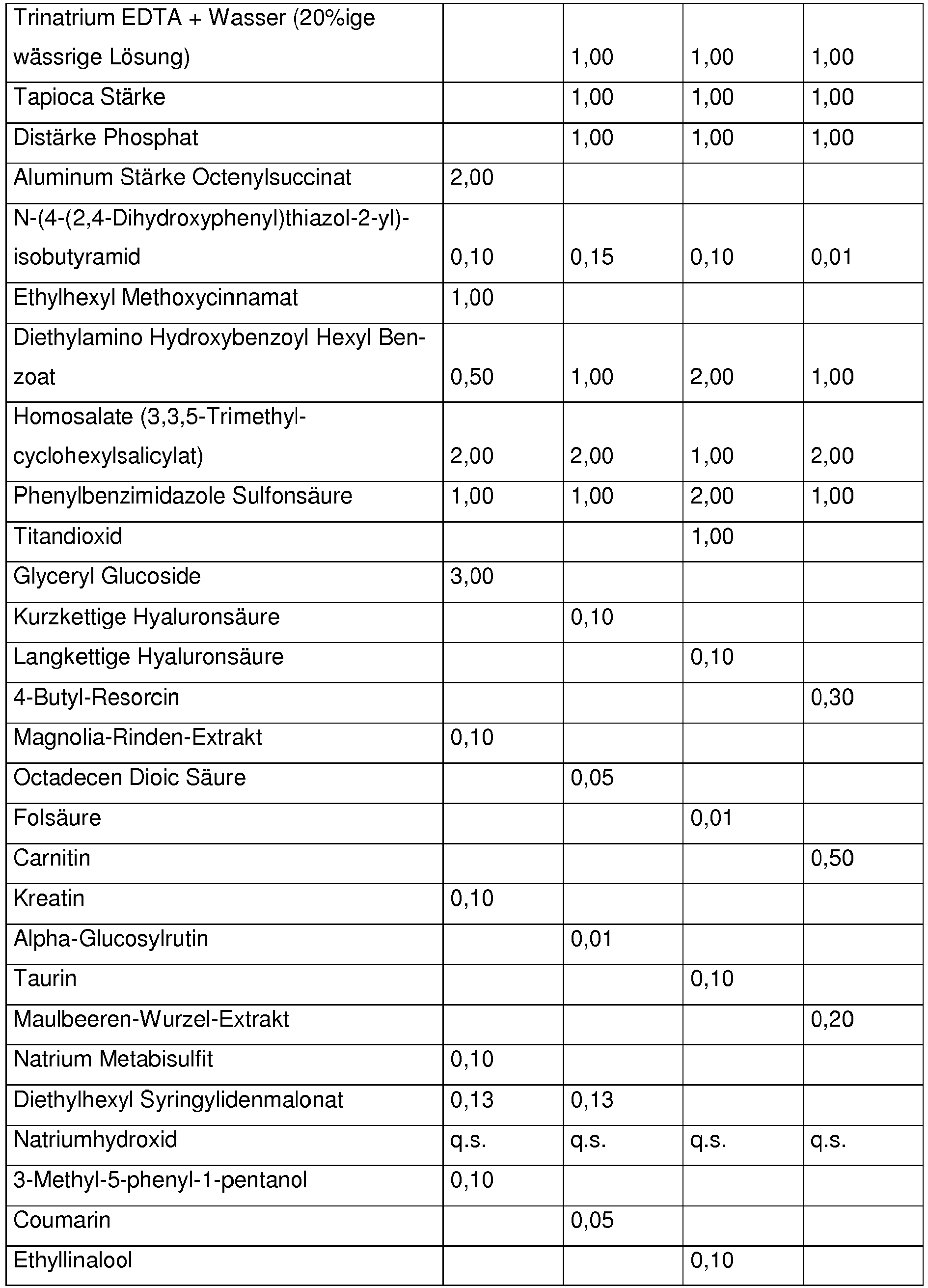

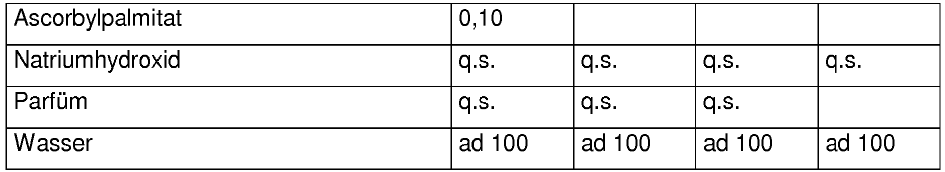

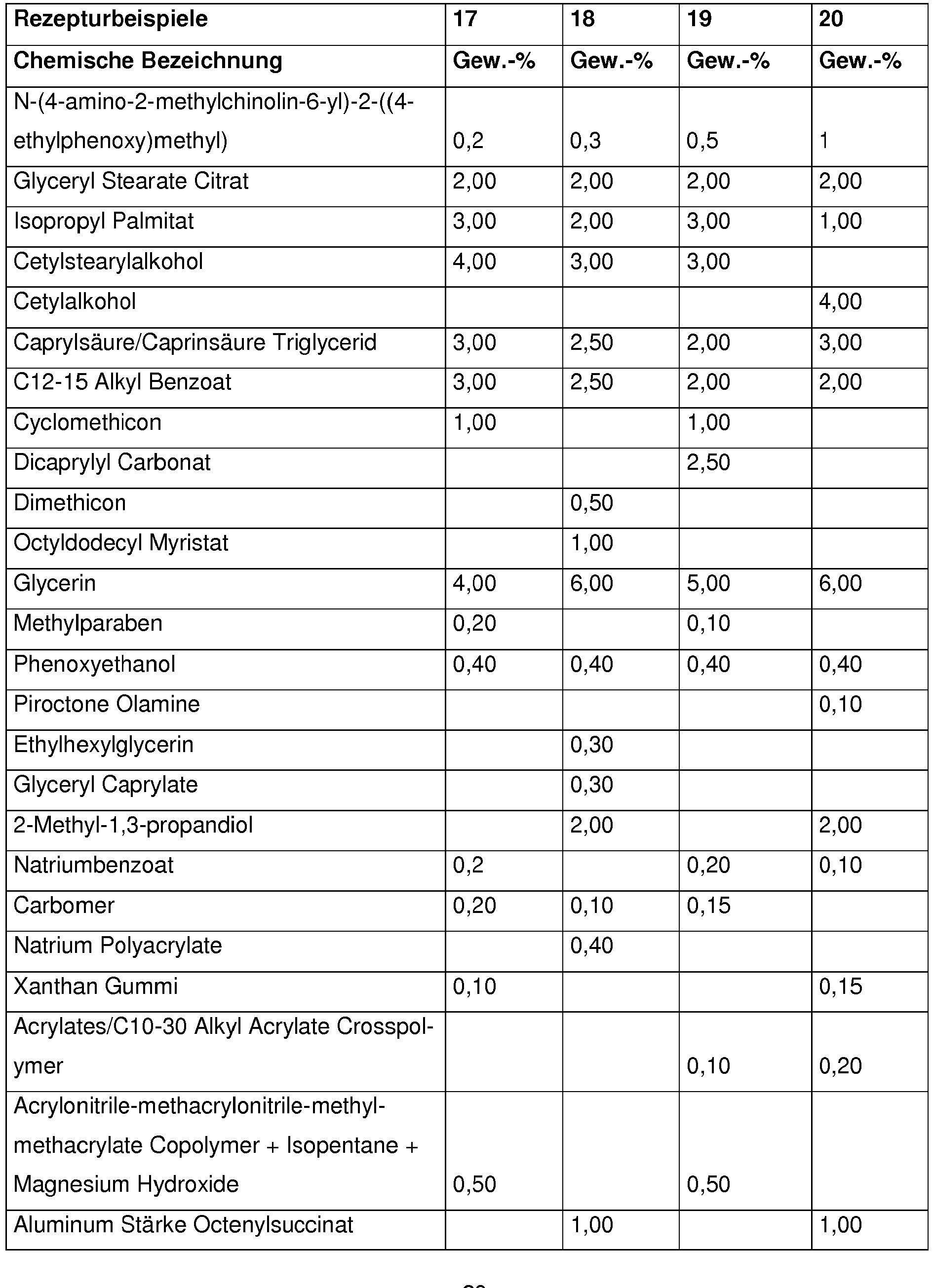

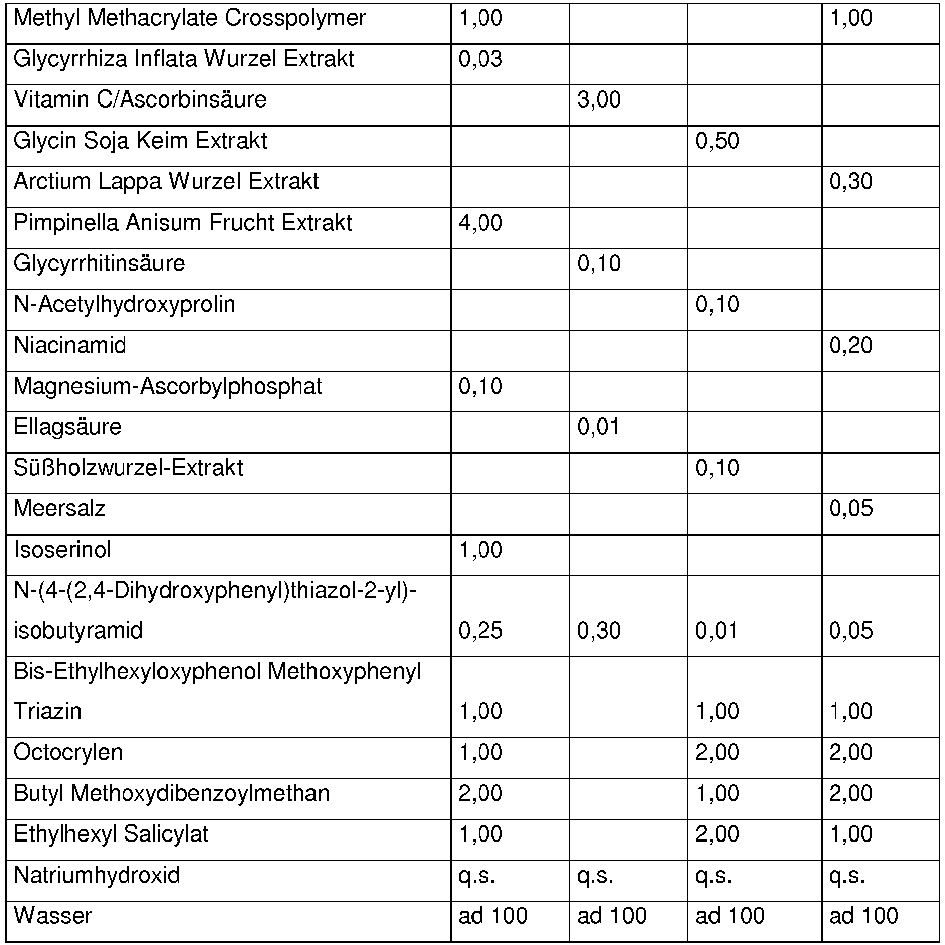

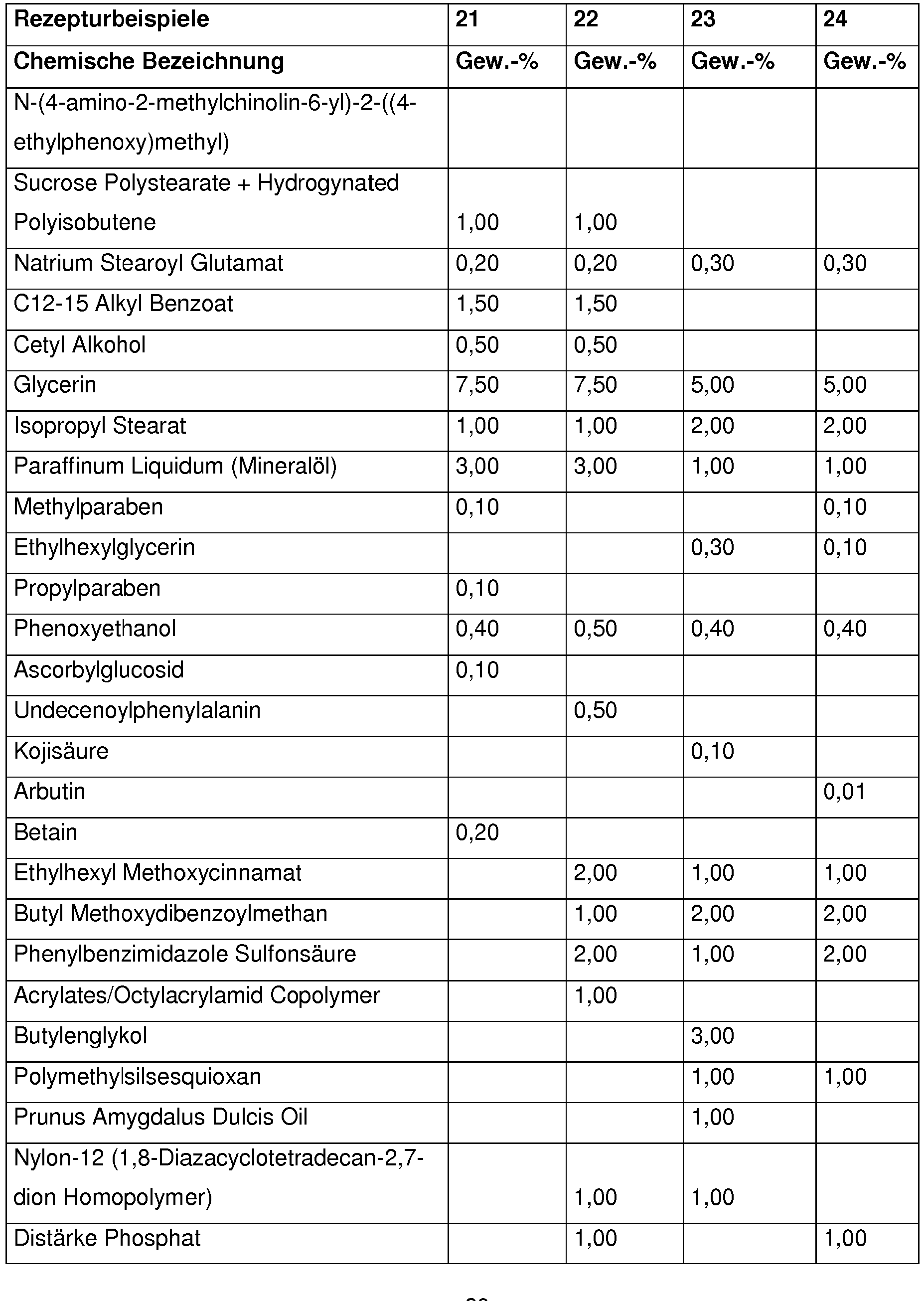

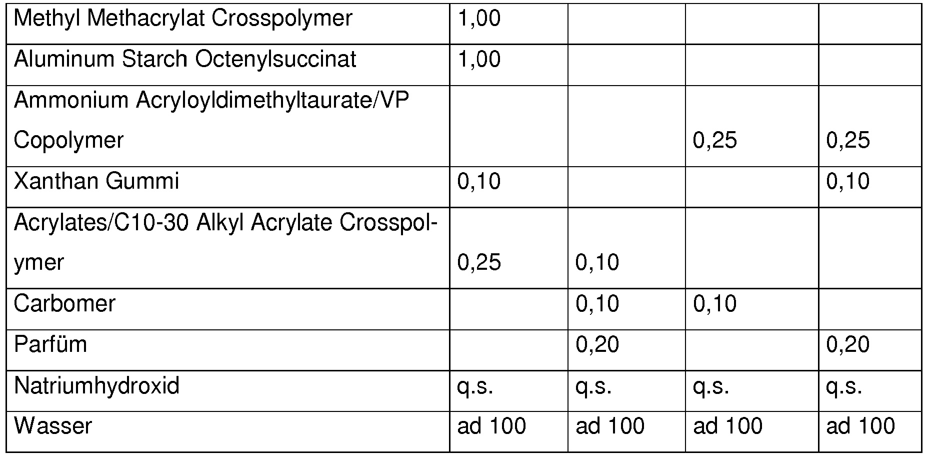

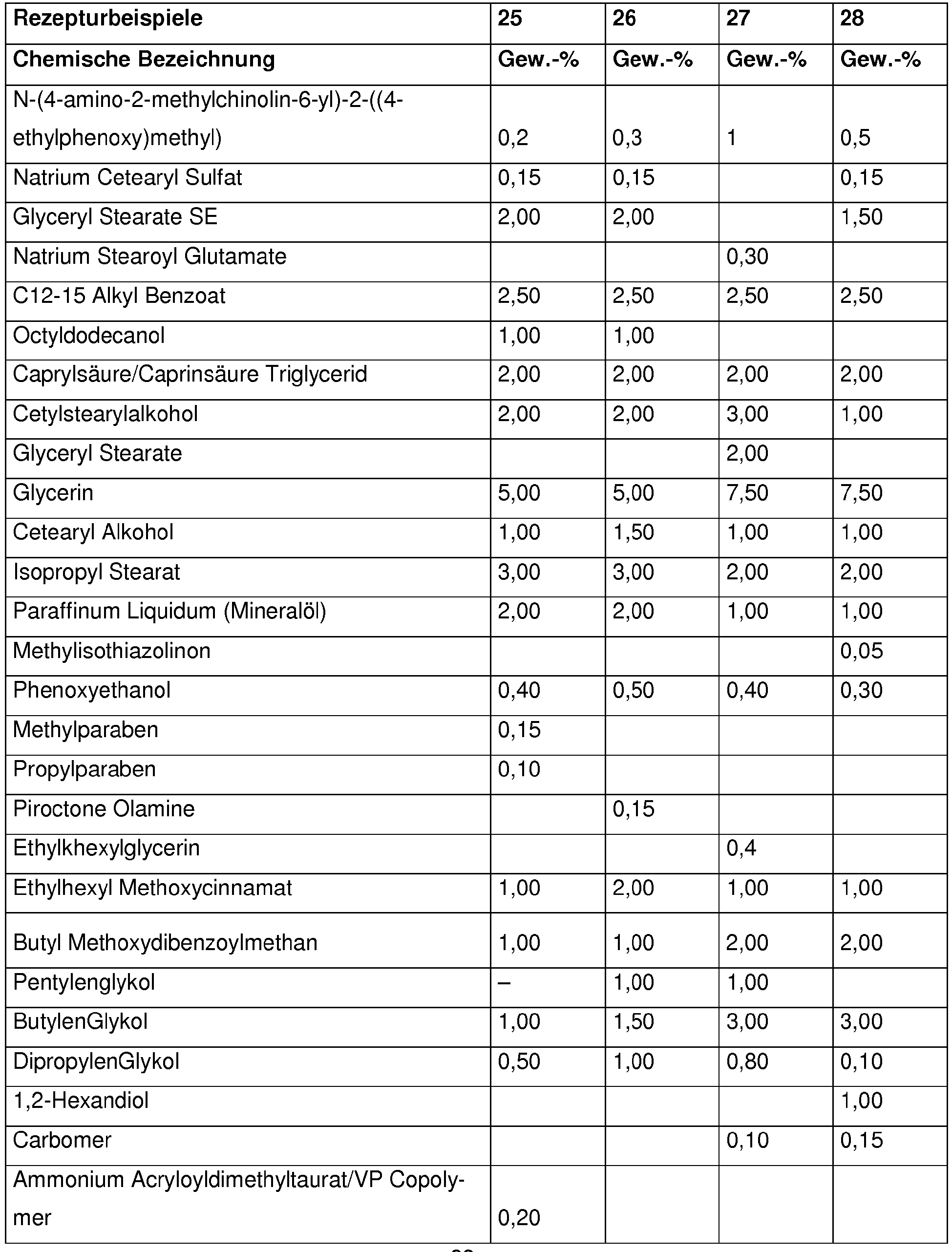

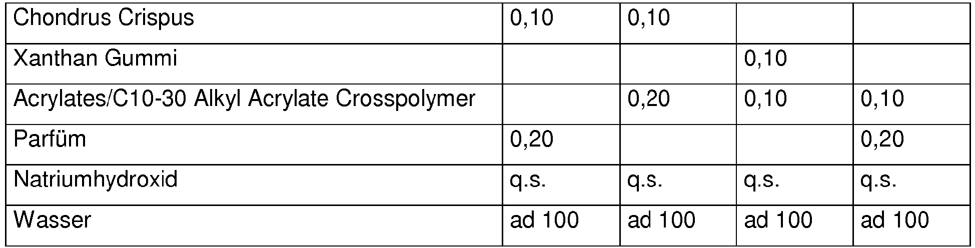

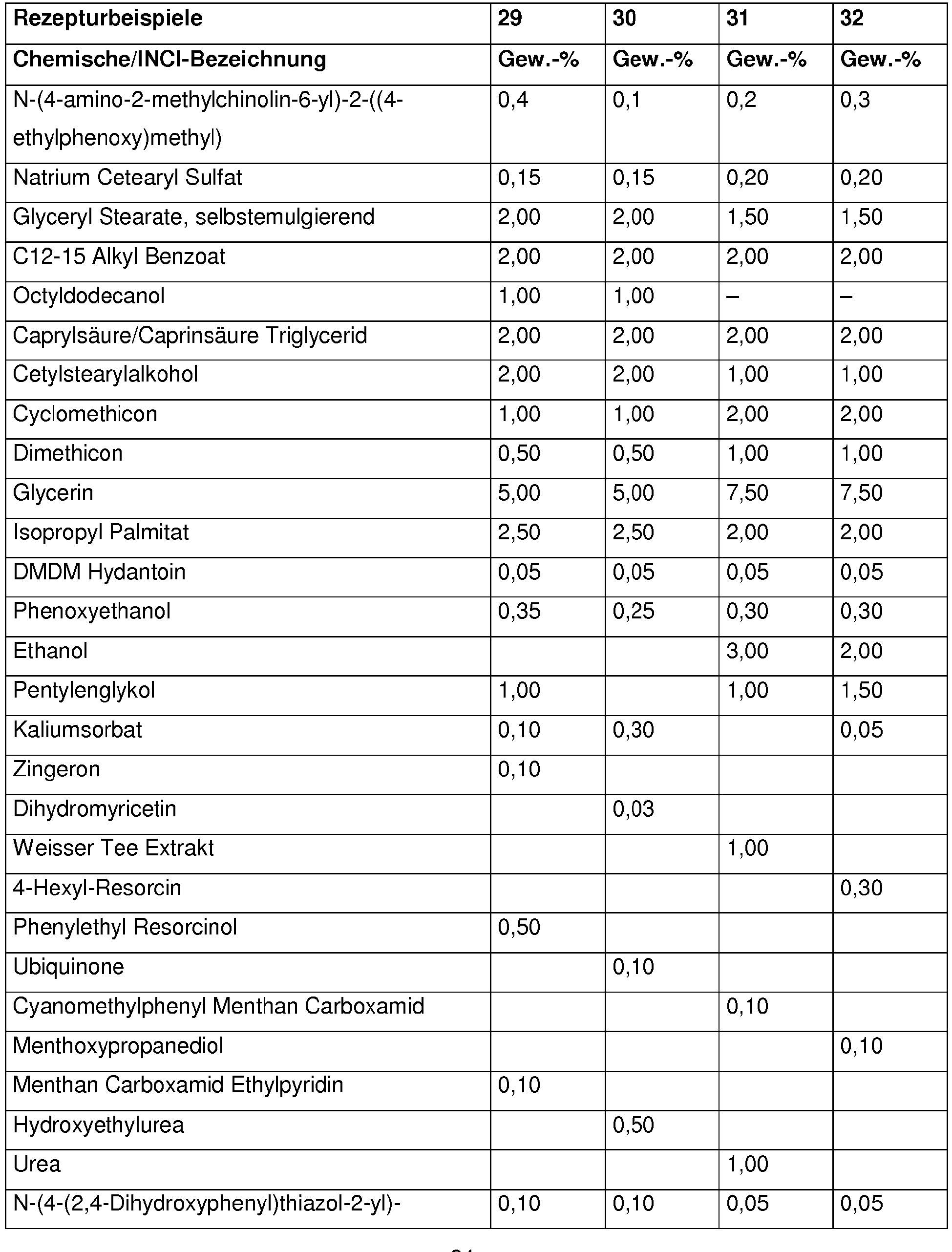

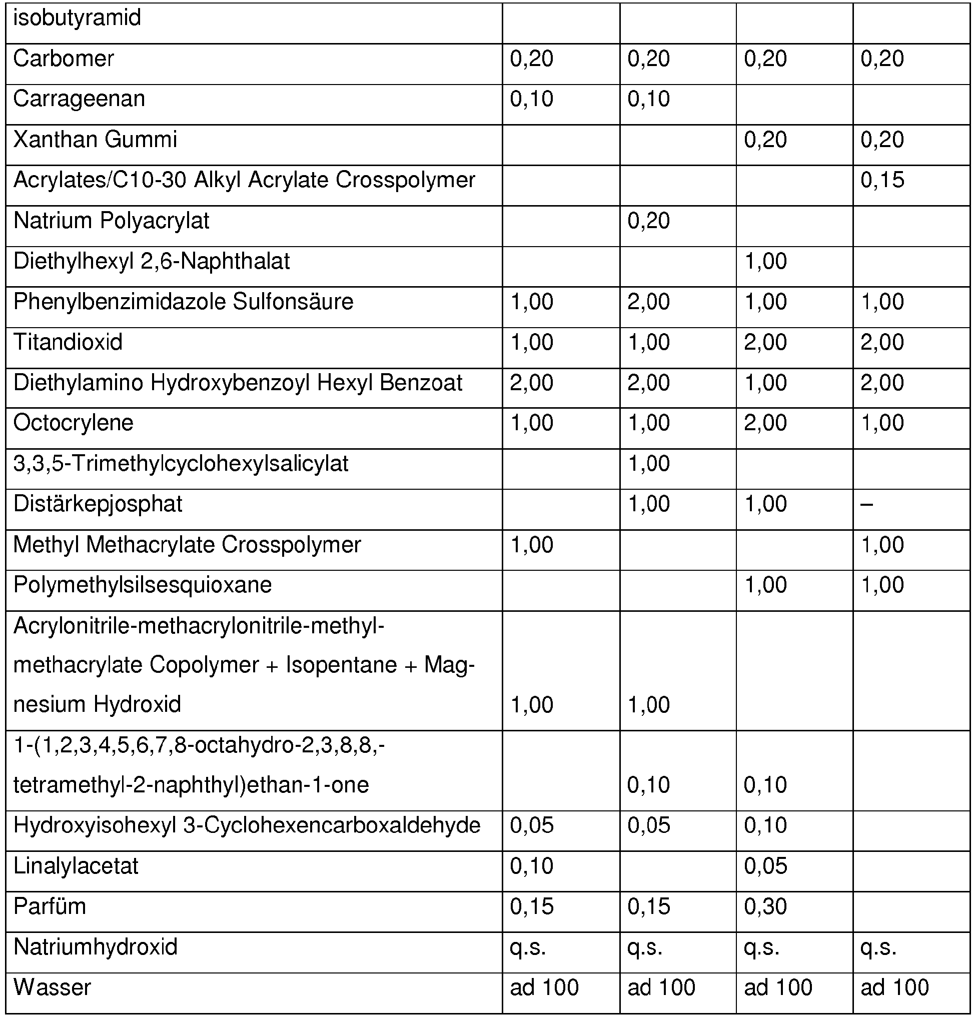

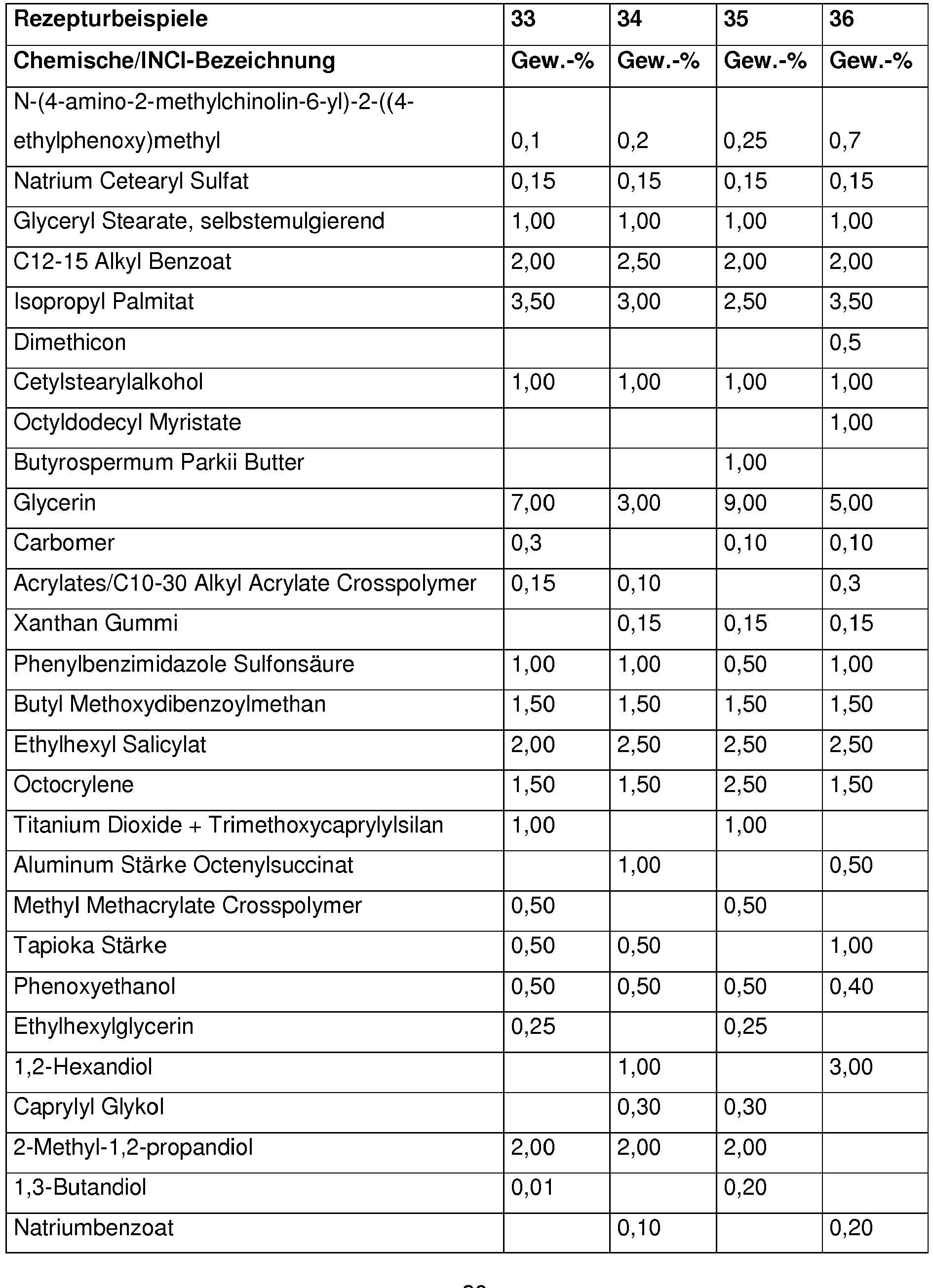

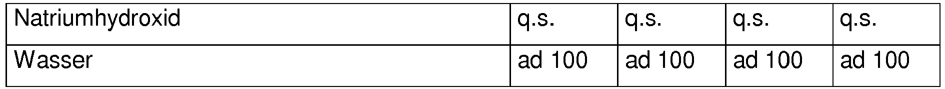

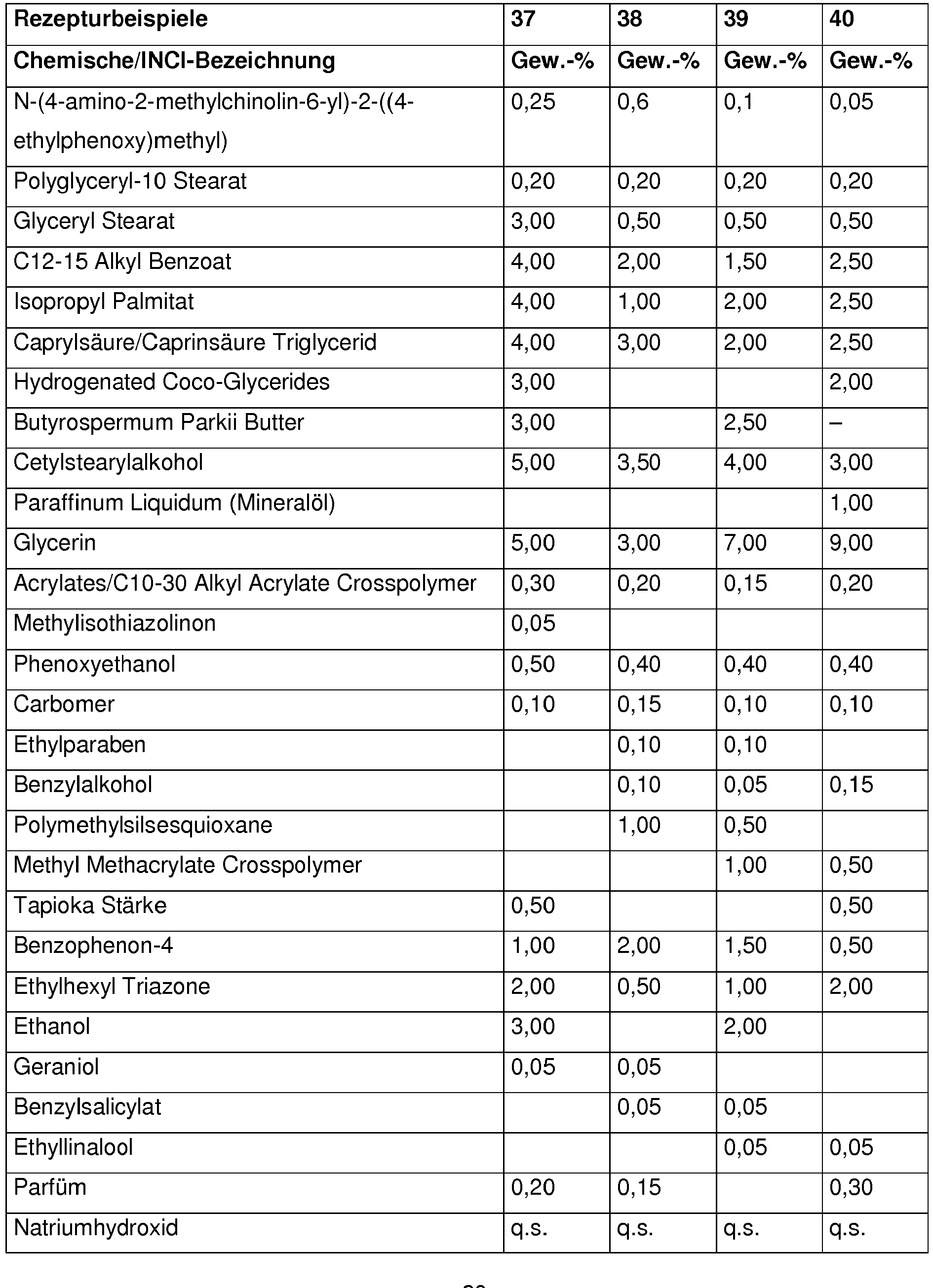

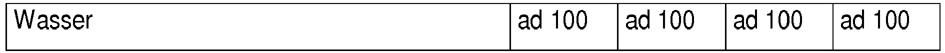

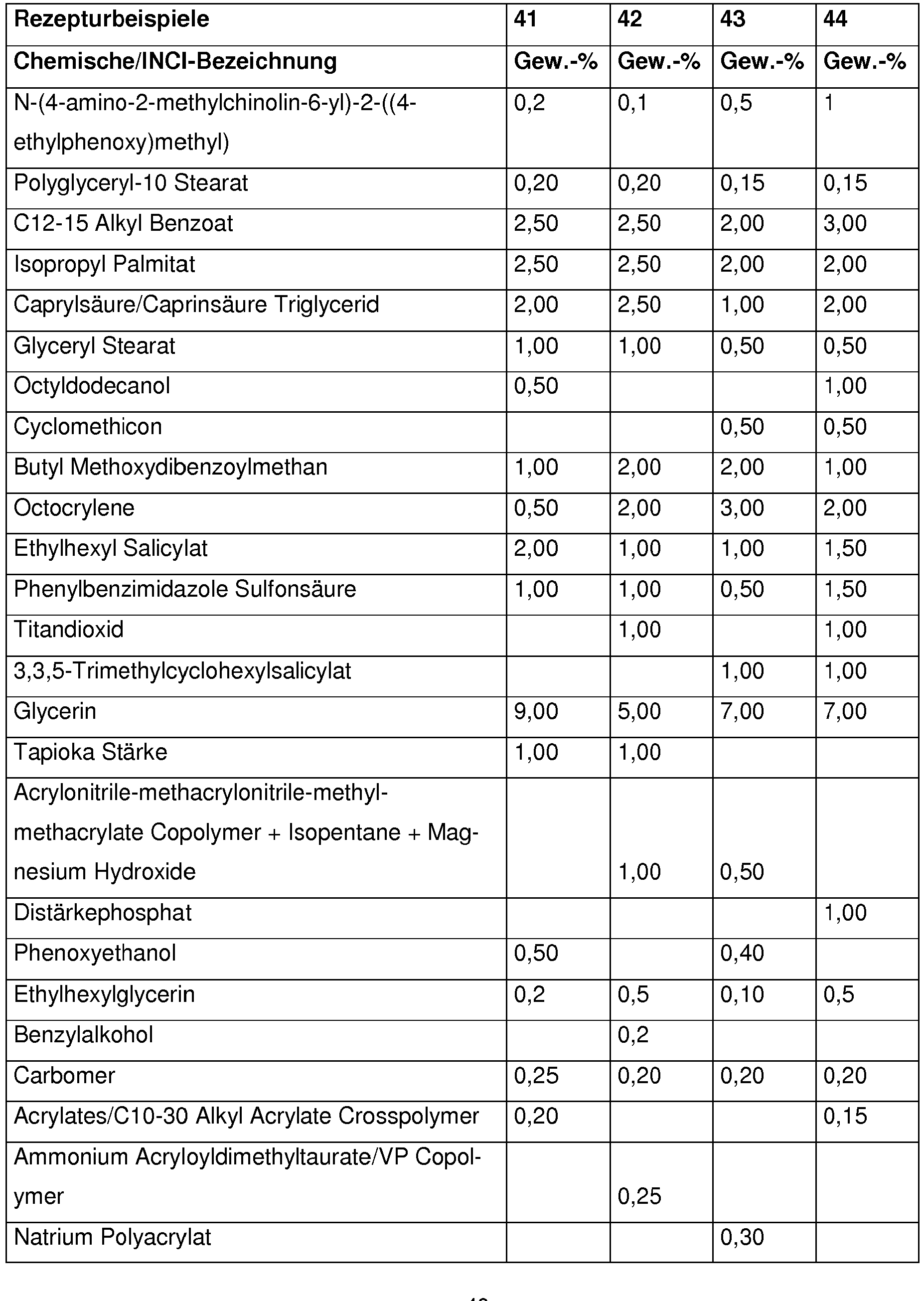

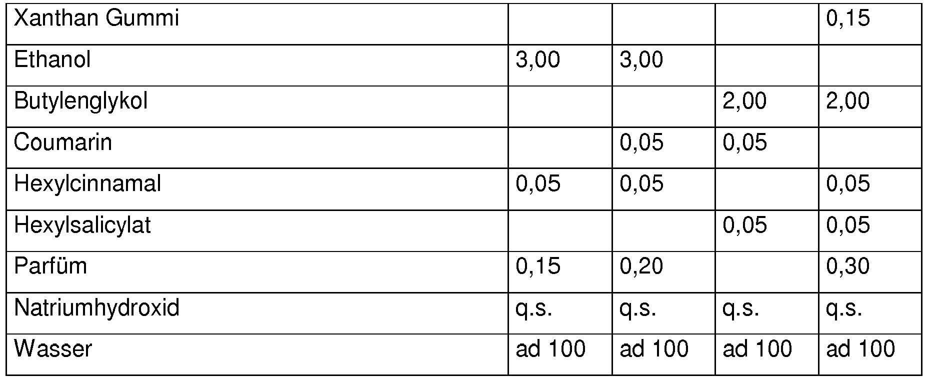

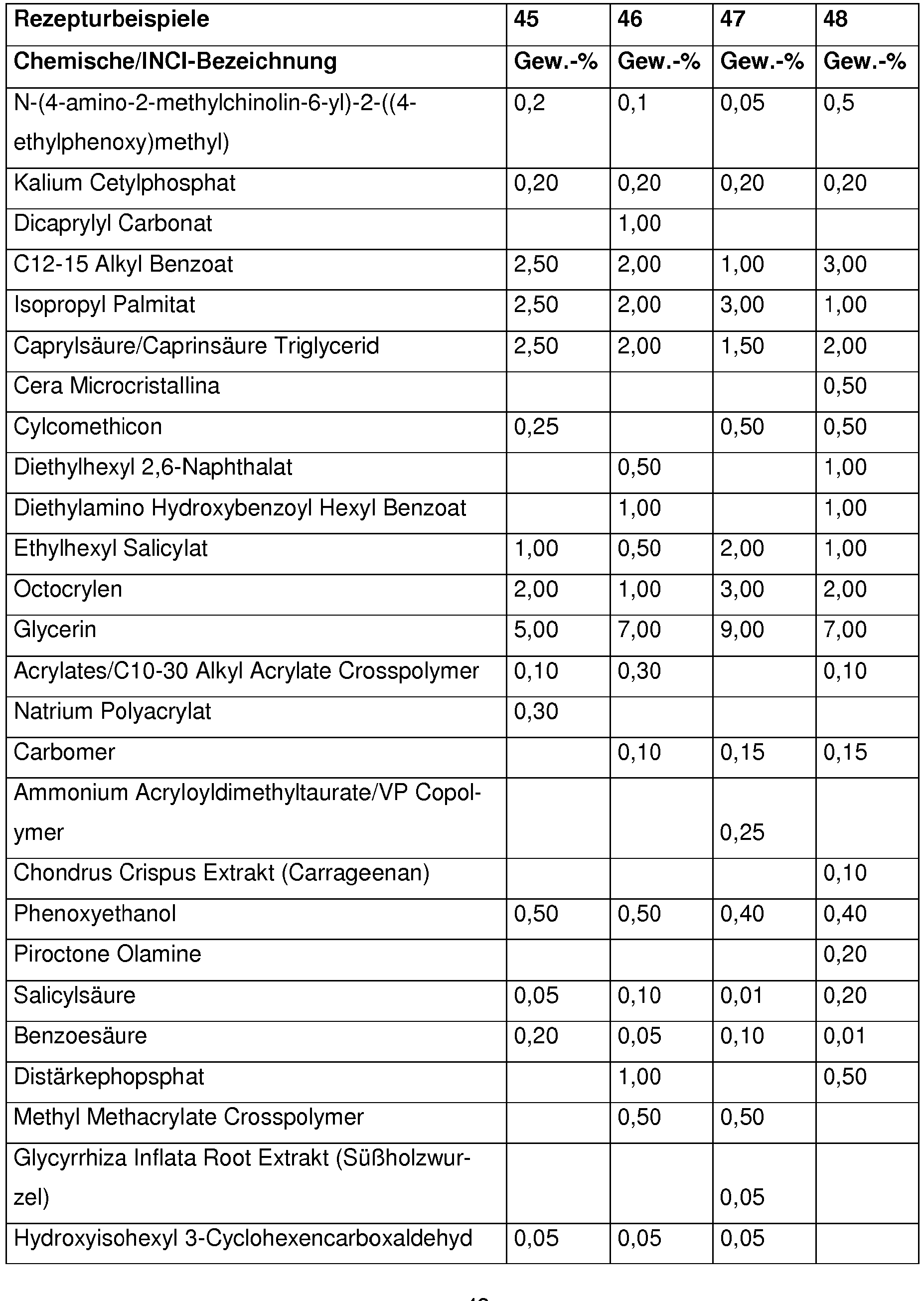

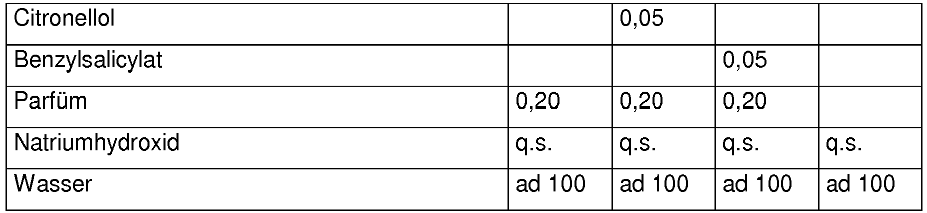

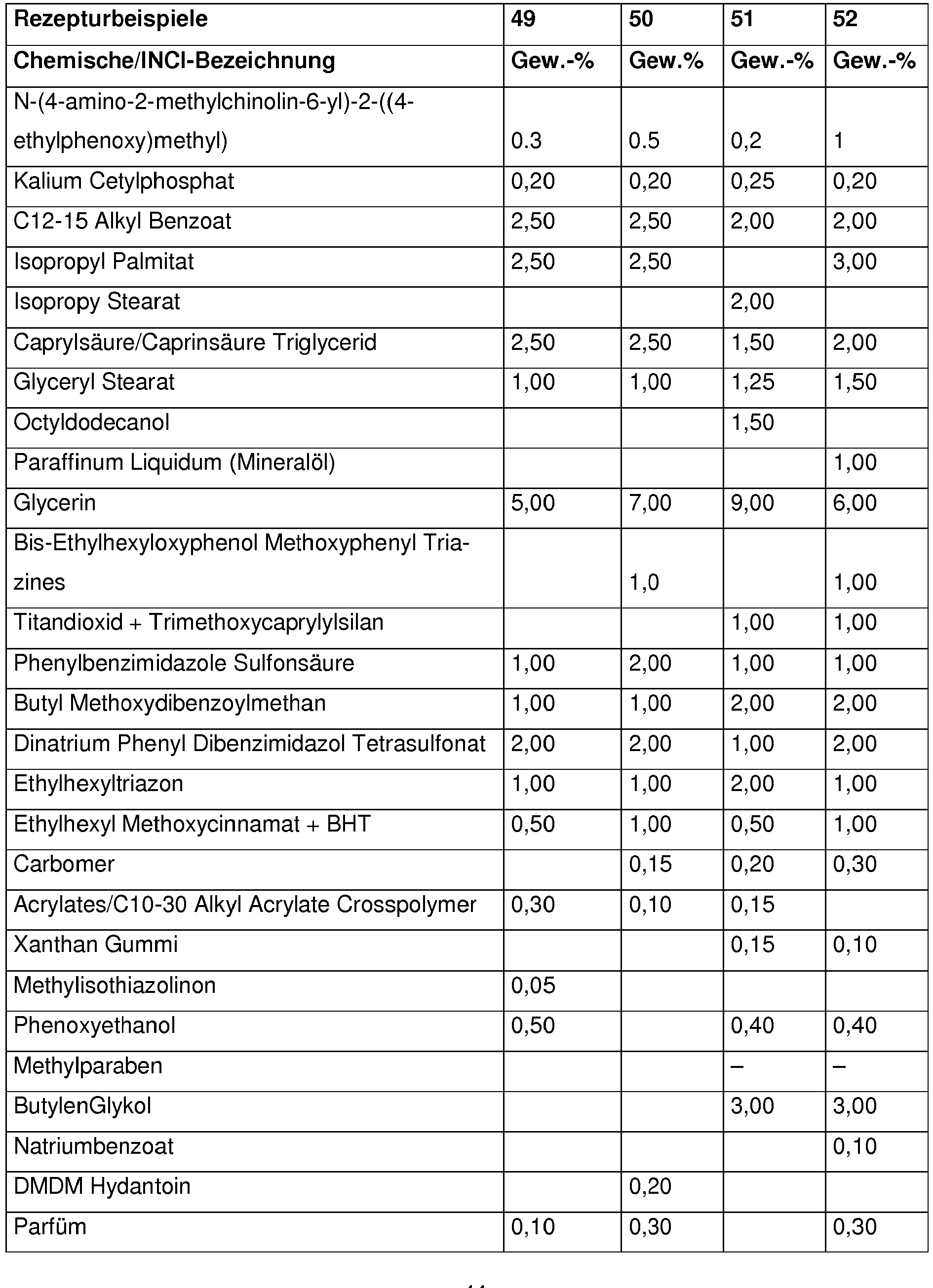

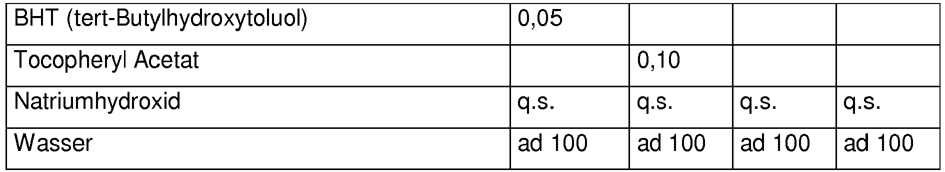

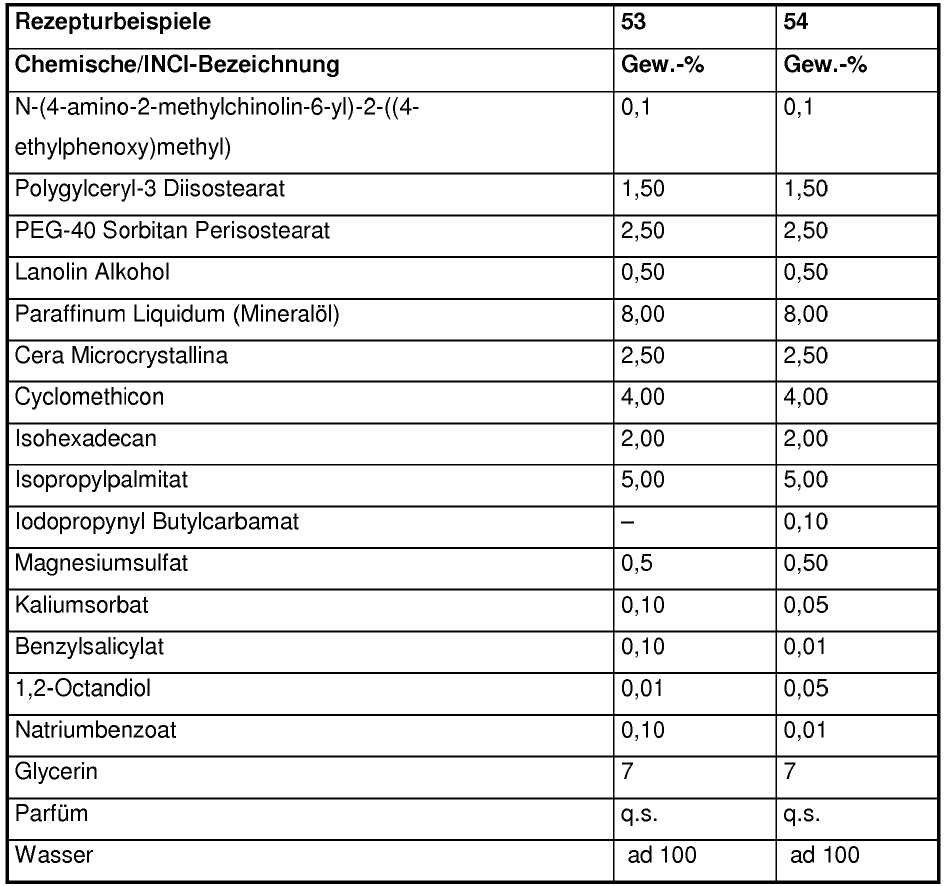

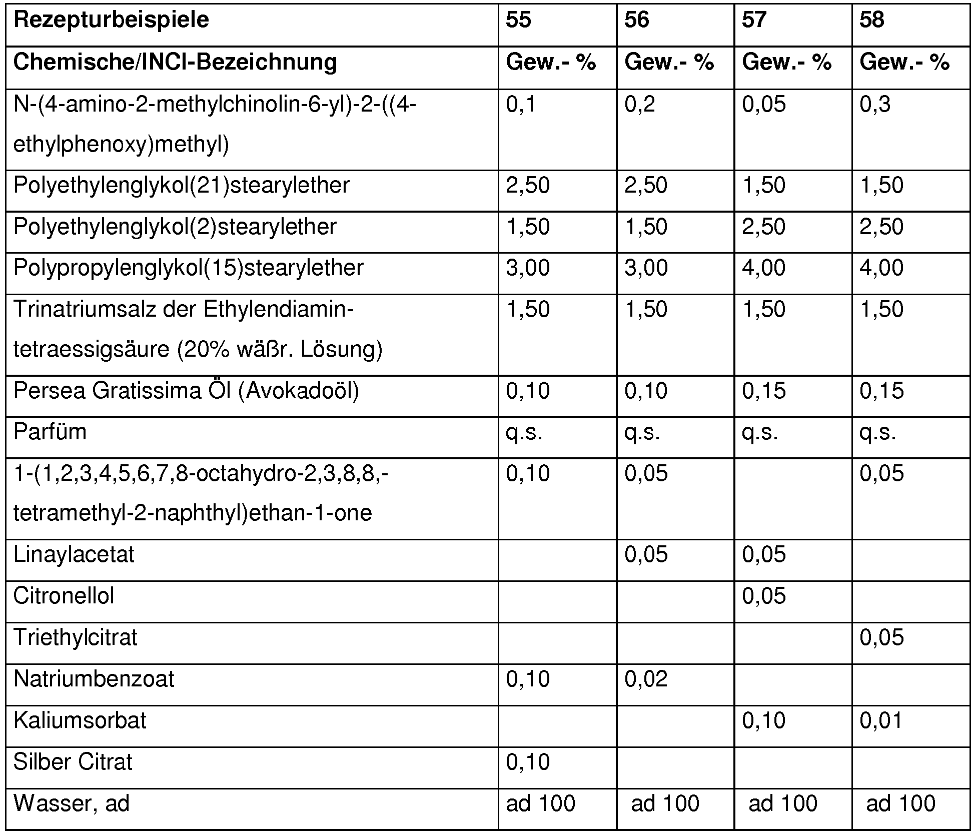

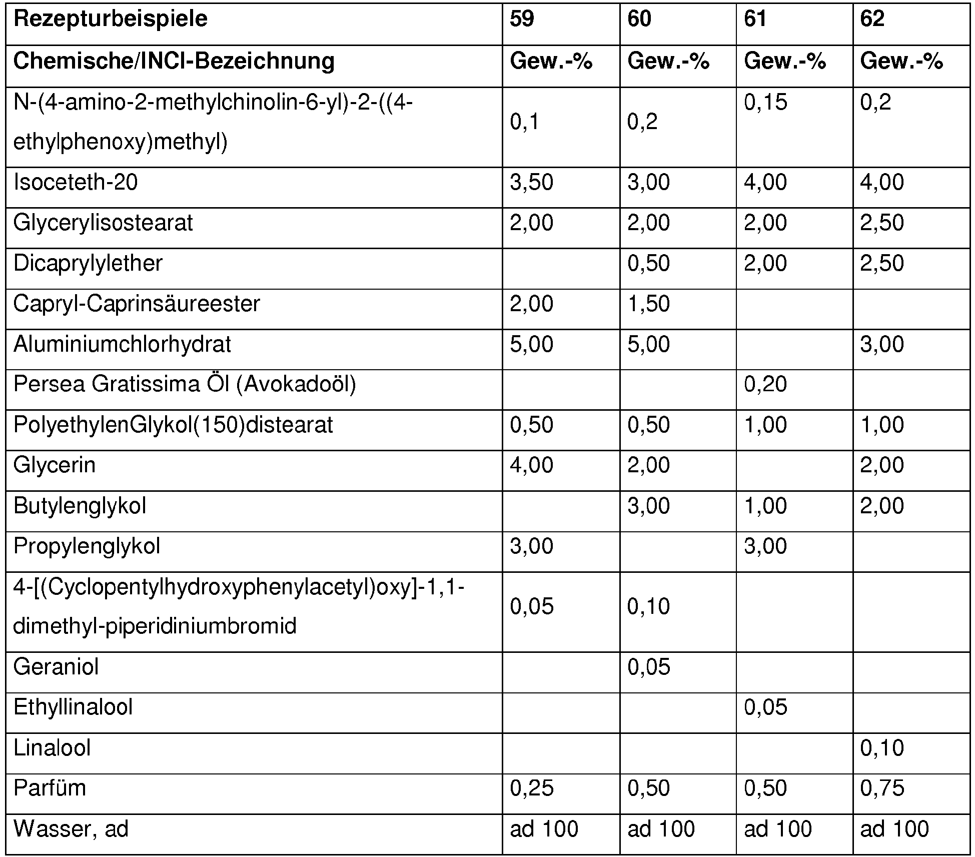

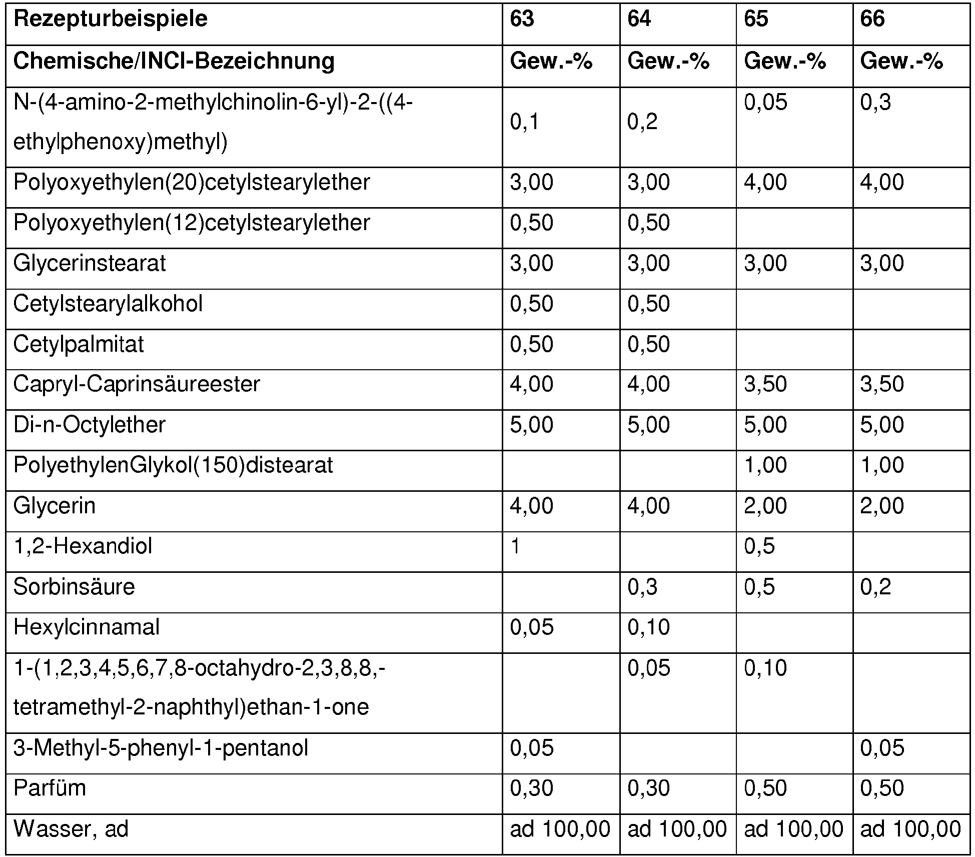

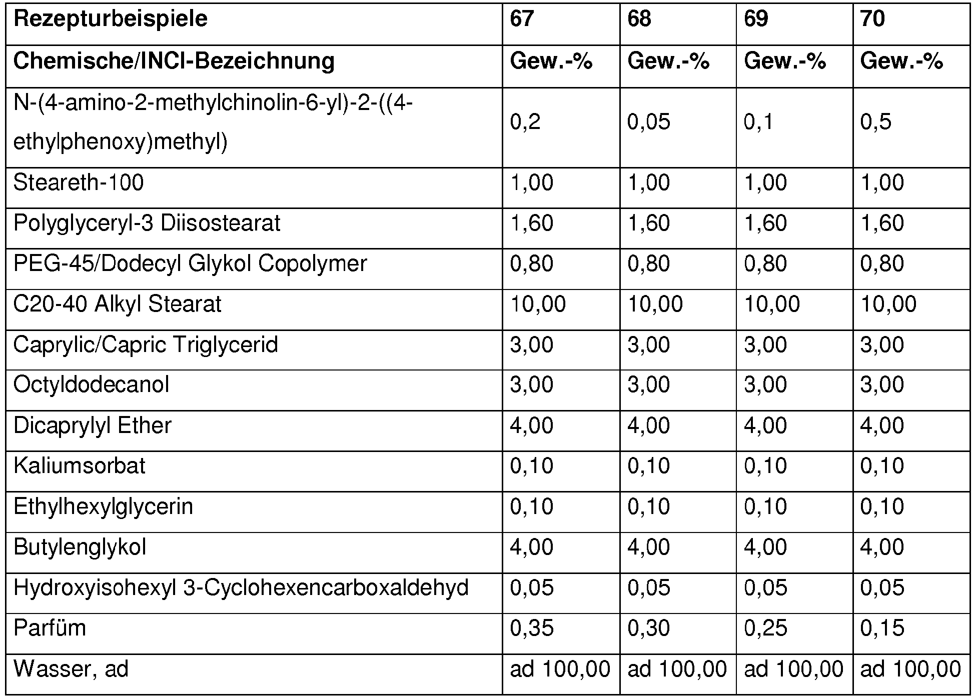

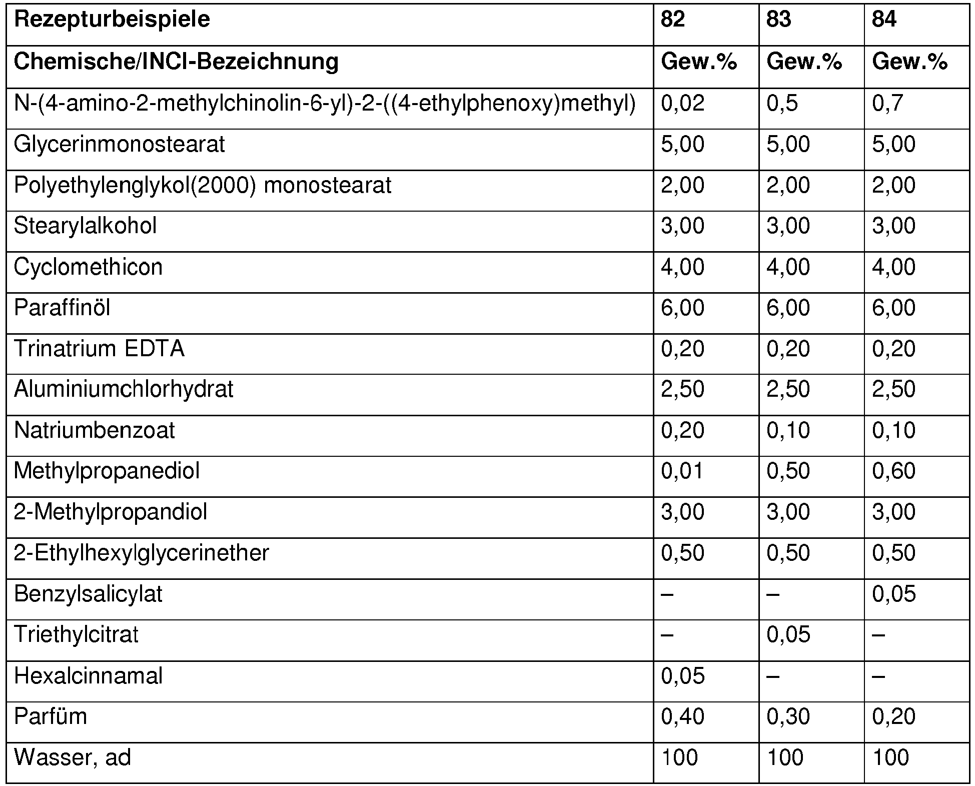

## Patentansprüche

1. Wirkstoffkombinationen aus N-(4-amino-2-methylchinolin-6-yl)-2-((4-ethylphenoxy)methyl) benzamid und einem oder mehreren kosmetisch oder dermatologisch unbedenklichen Konservierungsmitteln und/oder Konservierungshelfern.

2. Wirkstoffkombinationen nach Anspruch 1, **dadurch gekennzeichnet, dass** der oder die Konservierungsmittel und/oder Konservierungshelfer gewählt wird oder werden aus der Gruppe-4-Hydroxybenzoesäuremethylester, Etylparaben , Phenoxyethanol ,Pirocton-Olamin, Methylisothiazolinon ,Methylpropanediol, Butylene Glycol, Caprylyl Glycol ,Octan-1,2-diol, Ethylhexylglycerin, Glyceryl Caprylate , Propyleneglycol , Natrium Salicylat, Natrium Benzoat,Pentylene Glycol ,1,2-Hexanediol, DMDM Hydantoin, Benzylalkohol, Butylene Glycol, lodopropynyl Butylcarbamate, Propylenglycol, Butylenglycol, 2-Methylpropan-1,3-diol, Ethylhexylglycerin, 1,2-Pentandiol, 1,2-Hexandiol, 1,2-Octandiol und/oder 1,2-Decandiol.

3. Kosmetische oder dermatologische Zubereitungen, enthaltend Wirkstoffkombinationen nach einem der vorstehenden Ansprüche.

4. Zubereitungen nach Anspruch 4, enthaltend 0,01 - 20 Gew.-% an einem oder mehreren Konservierungsmittel und/oder Konservierungshelfer, **dadurch gekennzeichnet, dass** der oder die Konserviezungsmittel und/oder Konservierungshelfer gewählt wird oder werden aus der Gruppe, vorzugsweise 0,1 - 5 Gew.-%, bezogen auf das Gesamtgewicht der Zubereitungen.

5. Zubereitungen nach Anspruch 4 oder 5, enthaltend 0,001 - 10 Gew.-% an N-(4-amino-2-methylchinolin-6-yl)-2-((4-ethylphenoxy)methyl)benzamid, bezogen auf das Gesamtgewicht der Zubereitungen.

6. Wirkstoffkombinationen oder Zubereitungen, solche Wirkstoffkombinationen enthaltend nach einem der vorstehenden Ansprüche, zur Verwendung in der Behandlung und/oder Prophylaxe der Symptome der intrinsischen und/oder extrinsischen Hautalterung sowie zur Behandlung und Prophylaxe der schädlichen Auswirkungen ultravioletter Strahlung auf die Haut.

## Claims

1. Active substance combination of N-(4-amino-2-methylquinolin-6-yl)-2-((4-ethylphenoxy)methyl)benzamide and one or more cosmetically or dermatologically acceptable preservatives and/or preservative auxiliaries.

2. Active substance combination according to Claim 1, **characterized in that** the one or more preservatives and/or preservative auxiliaries are selected from the group consisting of methyl 4-hydroxybenzoate, ethylparaben, phenoxyethanol, piroctone olamine, methylisothiazolinone, methylpropanediol, butylene glycol, caprylyl glycol, octane-1,2-diol, ethylhexylglycerol, glyceryl caprylate, propylene glycol, sodium salicylate, sodium benzoate, pentylene glycol, 1,2-hexanediol, DMDM hydantoin, benzyl alcohol, butylene glycol, iodopropynyl butylcarbamate, propylene glycol, butylene glycol, 2-methylpropane-1,3-diol, ethylhexylglycerol, pentane-1,2-diol, hexane-1,2-diol, octane-1,2-diol, and/or decane-1,2-diol.

3. Cosmetic or dermatological preparation comprising an active substance combination according to either of the preceding claims.

4. Preparations according to Claim 4, containing 0.01-20% by weight of one or more preservatives and/or preservative auxiliaries, **characterized in that** the one or more preservatives and/or preservative auxiliaries are selected from the group, preferably 0.1-5% by weight based on the total weight of the preparations.

5. Preparations according to Claim 4 or 5, containing 0.001-10% by weight of N-(4-amino-2-methylquinolin-6-yl)-2-((4-ethylphenoxy)methyl)benzamide based on the total weight of the preparations.

6. Active substance combinations or preparations comprising such active substance combinations according to any of the preceding claims, for use in the treatment and/or prophylaxis of the symptoms of intrinsic and/or extrinsic skin aging and for the treatment and prophylaxis of the harmful effects on the skin of ultraviolet radiation.

## Revendications

1. Combinaisons de principes actifs composées de N-(4-amino-2-méthylquinoléin-6-yl)-2-((4-éthylphénoxy)méthyl)benzamide et d'un ou plusieurs agents de conservation et/ou auxiliaires de conservation acceptables sur le plan cosmétique ou dermatologique.

2. Combinaisons de principes actifs selon la revendication 1, **caractérisées en ce que** le ou les agents de conservation et/ou auxiliaires de conservation est ou sont choisi(s) dans le groupe de l'ester méthylique de l'acide 4-hydroxybenzoïque, de l'éthylparabène, du phénoxyéthanol, de la piroctone olamine, de la méthylisothiazolinone, du méthylpropanediol, du butylèneglycol, du caprylylglycol, de l'octane-1,2-diol, de l'éthylhexylglycérine, du caprylate de glycéryle, du propylèneglycol, du salicylate de sodium, du benzoate de sodium, du pentylèneglycol, du 1,2-hexanediol, de l'hydantoïne DMDM, de l'alcool benzylique, du butylèneglycol, du butylcarbamate d'iodopropynyle, du propylèneglycol, du butylèneglycol, du 2-méthylpropane-1,3-diol, de l'éthylhexylglycérine, du 1,2-pentanediol, du 1,2-hexanediol, du 1,2-octanediol et/ou du 1,2-décanediol.

3. Préparations cosmétiques et/ou dermatologiques, contenant des combinaisons de principes actifs selon l'une quelconque des revendications précédentes.

4. Préparations selon la revendication 4, contenant 0,01 à 20 % en poids d'un ou plusieurs agents de conservation et/ou auxiliaires de conservation, **caractérisées en ce que** le ou les agents de conservation et/ou auxiliaires de conservation est ou sont choisi(s) dans le groupe, de préférence 0,1 à 5 % en poids, par rapport au poids total des préparations.

5. Préparations selon la revendication 4 ou 5, contenant 0,001 à 10 % en poids de N-(4-amino-2-méthylquinoléin-6-yl)-2-((4-éthylphénoxy)méthyl)benzamide, par rapport au poids total des préparations.

6. Combinaisons de principes actifs ou préparations, contenant de telles combinaisons de principes actifs, selon l'une quelconque des revendications précédentes, pour une utilisation dans le traitement et/ou la prophylaxie des symptômes du vieillissement intrinsèque et/ou extrinsèque de la peau ainsi que pour le traitement et la prophylaxie des effets nuisibles d'un rayonnement ultraviolet sur la peau.
